(19) Europäisches Patentamt
European Patent Office
Office européen des brevets

(11) Veröffentlichungsnummer : **0 443 980 A1**

(12) **EUROPÄISCHE PATENTANMELDUNG**

(21) Anmeldenummer : **91810032.2**

(22) Anmeldetag : **16.01.91**

(51) Int. Cl.[5] : **C07D 417/06**, C07D 409/06, C07D 411/06, C07D 405/06, A01N 43/653, A01N 43/50, A01N 43/72

(30) Priorität : **25.01.90 CH 241/90**

(43) Veröffentlichungstag der Anmeldung : **28.08.91 Patentblatt 91/35**

(84) Benannte Vertragsstaaten : **AT BE CH DE DK ES FR GB IT LI NL**

(71) Anmelder : **CIBA-GEIGY AG**
**Klybeckstrasse 141**
**CH-4002 Basel (CH)**

(72) Erfinder : **Riebli, Peter**
**Bienenheim**
**CH-6074 Giswil (CH)**
Erfinder : **Hubele, Adolf, Dr.**
**Obere Egg 9**
**CH-4312 Magden (CH)**

(54) **Mikrobizide.**

(57) Neue Phenyl-ether-azol-Derivate der Formel

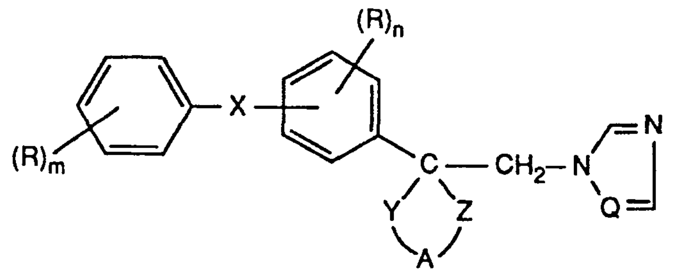

in welcher bedeuten :

R unabhängig voneinander Halogen, $C_1$-$C_4$-Alkyl, $C_1$-$C_3$-Alkoxy, $C_1$-$C_3$-Haloalkyl, $C_1$-$C_3$-Haloalkoxy, $NO_2$ oder CN ;

m 0 bis 5 ;

n 0 bis 4 ;

X und Y unabhängig voneinander Sauerstoff oder Schwefel ;

Z Schwefel, N-$R_a$ oder 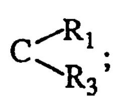 ;

$R_a$ Wasserstoff oder $C_1$-$C_6$-Alkyl ;

Q N oder CH ;

A das Ringglied 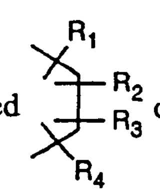 oder 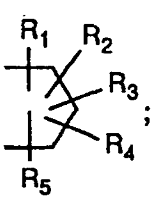 ;

$R_1$ und $R_2$ unabhängig voneinander Wasserstoff, Halogen, Hydroxy, $C_1$-$C_6$Alkyl, ein- oder mehrfach durch Halogen substituiertes $C_1$-$C_6$-Alkyl, oder $C_1$-$C_3$-Alkoxy oder ein- oder mehrfach durch Halogen substituiertes $C_1$-$C_3$-Alkoxy oder $CH_2X$-$R_6$ oder $C_3$-$C_6$-Cycloalkyl ;

$R_6$ Wasserstoff, $C_1$-$C_3$-Alkyl, $C_3$-$C_4$-Alkenyl, 2-Propinyl, 3-Halogen-2-propinyl, ein- oder mehrfach durch Halogen und/oder $C_1$-$C_2$-Alkoxy substituiertes $C_1$-$C_3$-Alkyl, durch $(R)_m$ substituiertes Phenyl oder durch $(R)_n$ oder $(R)_m$ substituiertes Benzyl ;

EP 0 443 980 A1

$R_3$, $R_4$ und $R_5$ unabhängig voneinander Wasserstoff, $C_1$-$C_3$-Alkyl ;

mit der Massgabe, dass falls Z Schwefel oder den Rest N-$R_a$ darstellt n $\geq$ 1 sein muss und falls Y Sauerstoff, Z den Rest $C(R_1)R_3$ und X Sauerstoff darstellen m $\geq$ 1 sein muss ; einschliesslich der Säureadditionssalze und Metallkomplexe der Verbindungen der vorstehenden Formel.

Die neuen Wirkstoffe besitzen pflanzenschützende Eigenschaften und eignen sich insbesondere zum Schutz von Pflanzen gegen den Befall von phytopathogenen Mikroorganismen wie Fungi, Bakterien und Viren.

# MIKROBIZIDE

Die vorliegende Erfindung betrifft neue substituierte Phenyl-ether-azol-Derivate der nach-stehenden Formel I sowie deren Säureadditionssalze und Metallkomplexe. Sie betrifft ferner die Herstellung dieser Verbindungen sowie agrochemische Mittel, die als Wirkstoff mindestens eine der Verbindungen der Formel I enthalten, die Herstellung dieser agro-chemischen Mittel und ihre Verwendung sowie ein Verfahren zur Bekämpfung oder Verhütung eines Befalls von Pflanzen durch phytopathogene Mikroorganismen.

Die erfindungsgemässen Verbindungen entsprechen der allgemeinen Formel I

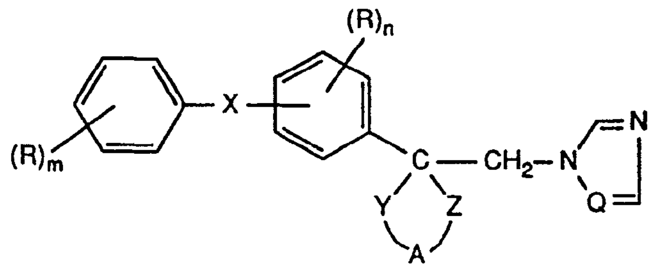 (I),

in welcher bedeuten :

R unabhängig voneinander Halogen, $C_1$-$C_4$-Alkyl, $C_1$-$C_3$-Alkoxy, $C_1$-$C_3$-Haloalkyl, $C_1$-$C_3$-Haloalkoxy, $NO_2$ oder CN ;

m 0 bis 5 ;

n 0 bis 4 ;

X und Y unabhängig voneinander Sauerstoff oder Schwefel ;

Z Schwefel, N-$R_a$ oder 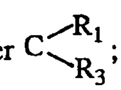 ;

$R_a$ Wasserstoff oder $C_1$ -$C_6$-Alkyl ;

Q N oder CH ;

A das Ringglied 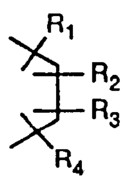 oder 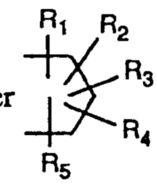 ;

$R_1$ und $R_2$ unabhängig voneinander Wasserstoff, Halogen, Hydroxy, $C_1$-$C_6$Alkyl, ein- oder mehrfach durch Halogen substituiertes $C_1$-$C_6$-Alkyl, oder $C_1$-$C_3$-Alkoxy oder ein- oder mehrfach durch Halogen substituiertes $C_1$-$C_3$-Alkoxy oder $CH_2X$-$R_6$ oder $C_3$-$C_6$-Cycloalkyl ;

$R_6$ Wasserstoff, $C_1$-$C_3$-Alkyl, $C_3$-$C_4$-Alkenyl, 2-Propinyl, 3-Halogen-2-propinyl, ein- oder mehrfach durch Halogen und/oder $C_1$-$C_2$-Alkoxy substituiertes $C_1$-$C_3$-Alkyl, durch $(R)_m$ substituiertes Phenyl oder durch $(R)_m$ substituiertes Benzyl ;

$R_3$, $R_4$ und $R_5$ unabhängig voneinander Wasserstoff, $C_1$-$C_3$-Alkyl ;

mit der Massgabe, dass falls Z Schwefel oder den Rest N-$R_a$ darstellt $n \geq 1$ sein muss und falls Y Sauerstoff, Z den Rest $C(R_1)R_3$ und X Sauerstoff darstellen $m \geq 1$ sein muss ; einschliesslich der Säureaddirionssalze und Metallkomplexe der Verbindungen der Formel I.

Unter dem Begriff Alkyl selbst oder als Bestandteil eines anderen Substituenten sind je nach Anzahl der angegebenen Kohlenstoffatome beispielsweise folgende Gruppen zu verstehen : Methyl, Ethyl, Propyl, Butyl, Pentyl oder Hexyl sowie z.B. ihre Isomeren Isopropyl, Isobutyl, tert.-Butyl, sek.-Butyl, Isopentyl usw.. Alkenyl steht z.B. für Propenyl-(1), Allyl, Butenyl-(1), Butenyl-(2) oder Butenyl-(3). Halogen soll hier und im folgenden Fluor, Chlor, Brom oder Jod, vorzugsweise Fluor, Chlor oder Brom, bedeuten.

Die Erfindung betrifft sowohl die freien Verbindungen der Formel I, als auch deren Additionssalze mit anorganischen und organischen Säuren, ebenso deren Komplexe mit Metallsalzen.

Erfindungsgemässe Salze sind insbesondere Additionssalze mit nach Massgabe des Einsatzzweckes physiologisch unbedenklichen anorganischen oder organischen Säuren, wie beispielsweise Halogenwasserstoffsäuren, z.B. Chlor-, Brom- oder Jodwasserstoffsäure, sowie Schwefelsäure, Phosphorsäure, phosphorige Säure, Salpetersäure, gegebenenfalls halogenierte Fettsäuren, wie Essigsäure, Trichloressigsäure und Oxalsäure, oder Sulfonsäuren, wie Benzolsulfonsäure und Methansulfonsäure oder auch Additionssalze mit geeigneten Salzen, wie beispielsweise Magnesium- oder Calciumchlorid.

Metallkomplexe der Formel I bestehen aus dem zugrundeliegenden organischen Molekül und einem anorganischen oder organischen Metallsalz, z.B. den Halogeniden, Nitraten, Sulfaten, Phosphaten, Tartraten usw. des Kupfers, Mangans, Eisens, Zinks und anderer Metalle. Dabei können die Metallkationen in den verschiedenen ihnen zukommenden Wertigkeiten vorliegen.

Die Verbindungen der Formel I sind bei Raumtemperatur stabile Oele, Harze oder Feststoffe, die sich durch sehr wertvolle physiologische, wie mikrobizide, beispielsweise pflanzenfungizide Eigenschaften auszeichnen. Sie lassen sich daher einerseits auf dem Agrargebiet oder verwandten Gebieten zur Bekämpfung von phytopathogenen Mikroorganismen einsetzen.

Aufgrund der besonderen mikrobiziden pflanzenschützenden Eigenschaften lassen sich die Verbindungen der Formel I in folgende Gruppen und Subgruppen einteilen, worin bedeuten :

1a) R unabhängig voneinander Halogen, $C_1$-$C_4$-Alkyl, $C_1$-$C_3$-Alkoxy, $C_1$-$C_3$-Haloalkyl, $C_1$-$C_3$-Haloalkoxy, $NO_2$ oder CN ;
m 0 bis 5 ;
n 1-4 ;
X und Y unabhängig voneinander Sauerstoff oder Schwefel ;
Z N-$R_a$ ;
$R_a$ Wasserstoff oder $C_1$-$C_6$-Alkyl ;
Q N oder CH ;

A das Ringglied 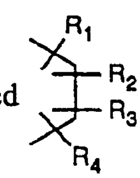 oder 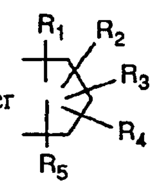 ;

$R_1$ und $R_2$ unabhängig voneinander Wasserstoff, $C_1$-$C_6$Alkyl, ein- oder mehrfach durch Halogen substituiertes $C_1$-$C_6$-Alkyl, oder $C_1$-$C_3$-Alkoxy oder $CH_2X$-$R_6$ oder $C_3$-$C_6$-Cycloalkyl ;
$R_6$ Wasserstoff, $C_1$-$C_3$-Alkyl, $C_3$-$C_4$-Alkenyl, 2-Propinyl, 3-Halogen-2-propinyl, ein- oder mehrfach durch Halogen und/oder $C_1$-$C_2$-Alkoxy substituiertes $C_1$-$C_3$-Alkyl, durch $(R)_m$ substituiertes Phenyl oder durch $(R)_m$ substituiertes Benzyl ;
$R_3$, $R_4$ und $R_5$ unabhängig voneinander Wasserstoff oder $C_1$-$C_3$-Alkyl ;
einschliesslich der Säureadditionssalze und Metallkomplexe.

1b) R unabhängig voneinander Halogen, $C_1$-$C_4$-Alkyl, Methoxy, $C_1$-$C_3$-Haloalkyl, $C_1$-$C_3$-Haloalkoxy ;
m 0-3 ;
n 0-2 ;
X Sauerstoff oder Schwefel ;
Y Schwefel ;
Z N-$R_a$ ;
$R_a$ Wasserstoff oder $C_1$-$C_6$-Alkyl ;
Q N oder CH ;

A das Ringglied 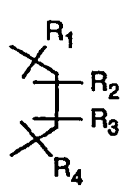 oder 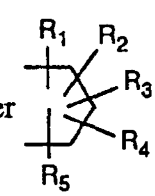 ;

$R_1$ und $R_2$ unabhängig voneinander Wasserstoff, $C_1$-$C_6$Alkyl, ein- oder mehrfach durch Halogen substituiertes $C_1$-$C_6$-Alkyl, oder $CH_2X$-$R_6$ ;
$R_6$ Wasserstoff, $C_1$-$C_3$-Alkyl, $C_3$-$C_4$-Alkenyl, 2-Propinyl, 3-Halogen-2-propinyl, ein- oder mehrfach durch

Halogen und/oder $C_1$-$C_2$-Alkoxy substituiertes $C_1$-$C_3$-Alkyl, oder durch $(R)_m$ substituiertes Benzyl ;
$R_3$, $R_4$ und $R_5$ unabhängig voneinander Wasserstoff oder $C_1$-$C_3$-Alkyl ;
einschliesslich der Säureadditionssalze und Metallkomplexe.
1c) R unabhängig voneinander Halogen, $C_1$-$C_2$-Alkyl, $CF_3$, $OCHF_2$ ;
m 0-2 ;
n 0-2 ;
X Sauerstoff oder Schwefel ;
Y Schwefel ;
Z N-$R_a$ ;
$R_a$ Wasserstoff oder $C_1$-$C_6$-Alkyl ;
Q N oder CH ;

A das Ringglied 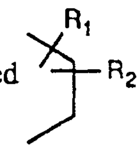 oder 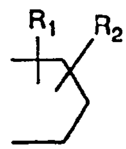 ;

$R_1$ und $R_2$ unabhängig voneinander Wasserstoff, $C_1$-$C_3$Alkyl, ein- oder mehrfach durch Halogen substituiertes $C_1$-$C_3$-Alkyl, oder $CH_2X$-$R_6$ ;
$R_6$ Wasserstoff, $C_1$-$C_3$-Alkyl, $C_3$-$C_4$-Alkenyl, 2-Propinyl oder 3-Halogen-2-propinyl ;
einschliesslich der Säureadditionssalze und Metallkomplexe.
1d) R unabhängig voneinander Halogen oder Methyl ;
m 0-2 ;
n 1 ;
X Sauerstoff ;
Y Schwefel ;
Z N-$R_a$ ;
$R_a$ Wasserstoff ;
Q N ;

A das Ringglied 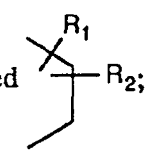 ;

$R_1$ und $R_2$ unabhängig voneinander Wasserstoff, $C_1$-$C_3$-Alkyl ; oder $CH_2O$-$R_6$ ;
$R_6$ Wasserstoff oder $C_1$-$C_3$-Alkyl ;
einschliesslich der Säureadditionssalze und Metallkomplexe.
2a) R unabhängig voneinander Halogen, $C_1$-$C_4$-Alkyl, $C_1$-$C_3$-Alkoxy, $C_1$-$C_3$-Haloalkyl, $C_1$-$C_3$-Haloalkoxy, $NO_2$ oder CN ;
m 0 bis 5 ;
n 1-4 ;
X und Y unabhängig voneinander Sauerstoff oder Schwefel ;
Z Schwefel ;
Q N oder CH ;

A das Ringglied 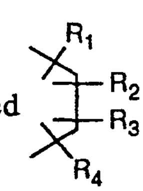 oder 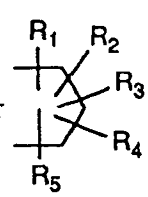 ;

$R_1$ und $R_2$ unabhängig voneinander Wasserstoff, $C_1$-$C_6$Alkyl, ein- oder mehrfach durch Halogen substituiertes $C_1$-$C_6$-Alkyl, oder $C_1$-$C_3$-Alkoxy oder $CH_2X$-$R_6$ oder $C_3$-$C_6$-Cycloalkyl ;

$R_6$ Wasserstoff, $C_1$-$C_3$-Alkyl, $C_3$-$C_4$-Alkenyl, 2-Propinyl, 3-Halogen-2-propinyl, ein- oder mehrfach durch Halogen und/oder $C_1$-$C_2$-Alkoxy substituiertes $C_1$-$C_3$-Alkyl, durch $(R)_m$ substituiertes Phenyl oder durch $(R)_m$ substituiertes Benzyl ;
$R_3$, $R_4$ und $R_5$ unabhängig voneinander Wasserstoff oder $C_1$-$C_3$-Alkyl ;
einschliesslich der Säureadditionssalze und Metallkomplexe.
2b) R unabhängig voneinander Halogen, $C_1$-$C_4$-Alkyl, $C_1$-$C_3$-Alkoxy, $C_1$-$C_3$-Haloalkyl oder $C_1$-$C_3$-Haloalkoxy ;
m 0-3 ;
n 1-2 ;
X und Y unabhängig voneinander Sauerstoff oder Schwefel ;
Z Schwefel ;
Q N oder CH ;

A das Ringglied 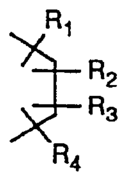 oder 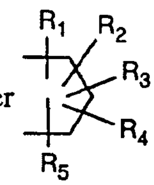 ;

$R_1$ und $R_2$ unabhängig voneinander Wasserstoff, $C_1$-$C_6$Alkyl, ein- oder mehrfach durch Halogen substituiertes $C_1$-$C_6$-Alkyl, oder $C_1$-$C_3$-Alkoxy oder $CH_2X$-$R_6$ ;
$R_6$ Wasserstoff, $C_1$-$C_3$-Alkyl, $C_3$-$C_4$-Alkenyl, 2-Propinyl, 3-Halogen-2-propinyl, ein- oder mehrfach durch Halogen und/oder $C_1$-$C_2$-Alkoxy substituiertes $C_1$-$C_3$-Alkyl, oder durch $(R)_m$ substituiertes Phenyl oder durch $(R)_m$ substituiertes Benzyl ;
$R_3$, $R_4$ und $R_5$ unabhängig voneinander Wasserstoff oder $C_1$-$C_3$-Alkyl ;
einschliesslich der Säureadditionssalze und Metallkomplexe.
2c) R unabhängig voneinander Halogen, $C_1$-$C_2$-Alkyl, Methoxy, $CF_3$ oder $OCHF_2$ ;
m 0-2 ;
n 1 ;
X und Y unabhängig voneinander Sauerstoff oder Schwefel ;
Z Schwefel ;
Q N oder CH ;

A das Ringglied 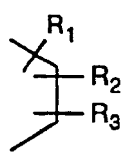 oder 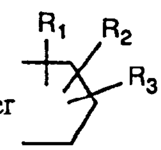 ;

$R_1$ und $R_2$ unabhängig voneinander Wasserstoff, $C_1$-$C_3$Alkyl, ein- oder mehrfach durch Halogen substituiertes $C_1$-$C_3$-Alkyl, oder $CH_2X$-$R_6$ ;
$R_6$ Wasserstoff, $C_1$-$C_3$-Alkyl, $C_3$-$C_4$-Alkenyl, 2-Propinyl oder 3-Halogen-2-propinyl ;
$R_3$ Wasserstoff oder $C_1$-$C_3$-Alkyl ;
einschliesslich der Säureadditionssalze und Metallkomplexe.
2d) R unabhängig voneinander Halogen oder Methyl ;
m 0 oder 1 ;
n 1 ;
X Sauerstoff ;
Y Sauerstoff oder Schwefel ;
Z Schwefel ;
Q N ;

A das Ringglied 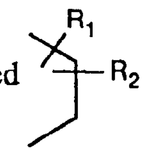 oder 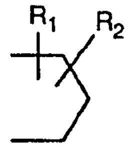 ;

$R_1$ und $R_2$ unabhängig voneinander Wasserstoff, $C_1$-$C_3$-Alkyl oder $CH_2O$-$R_6$ ; oder
$R_6$ Wasserstoff oder $C_1$-$C_3$-Alkyl ;
einschliesslich der Säureadditionssalze und Metallkomplexe.
3a) R unabhängig voneinander Halogen, $C_1$-$C_4$-Alkyl, $C_1$-$C_3$-Alkoxy, $C_1$-$C_3$-Haloalkyl, $C_1$-$C_3$-Haloalkoxy, $NO_2$ oder CN ;
m 0 bis 5 ;
n 0 bis 4 ;
X Sauerstoff oder Schwefel ;
Y Schwefel ;

Z 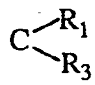 ;

Q N oder CH ;

A das Ringglied 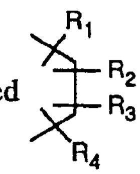 oder 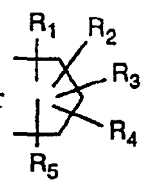 ;

$R_1$ und $R_2$ unabhängig voneinander Wasserstoff, Halogen, Hydroxy, $C_1$-$C_6$Alkyl, ein- oder mehrfach durch Halogen substituiertes $C_1$-$C_6$-Alkyl, oder $C_1$-$C_3$-Alkoxy oder ein- oder mehrfach durch Halogen substituiertes $C_1$-$C_3$-Alkoxy oder $CH_2X$-$R_6$ oder $C_3$-$C_6$-Cycloalkyl ;
$R_6$ Wasserstoff, $C_1$-$C_3$-Alkyl, $C_3$-$C_4$-Alkenyl, 2-Propinyl, 3-Halogen-2-propinyl, ein- oder mehrfach durch Halogen und/oder $C_1$-$C_2$-Alkoxy substituiertes $C_1$-$C_3$-Alkyl, durch $(R)_m$ substituiertes Phenyl oder durch $(R)_m$ substituiertes Benzyl ;
$R_3$, $R_4$ und $R_5$ unabhängig voneinander Wasserstoff oder $C_1$-$C_3$-Alkyl ;
einschliesslich der Säureadditionssalze und Metallkomplexe.
3b) R unabhängig voneinander Halogen, $C_1$-$C_4$-Alkyl, Methoxy, $CF_3$ oder $OCHF_2$ ;
m 0 bis 3 ;
n 0 bis 2 ;
X Sauerstoff oder Schwefel ;
Y Schwefel ;

Z 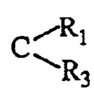 ;

Q N oder CH ;

A das Ringglied 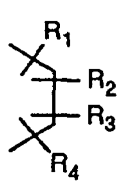 oder 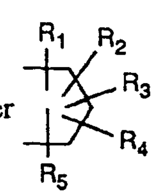 ;

$R_1$ und $R_2$ unabhängig voneinander Wasserstoff, Halogen, Hydroxy, $C_1$-$C_3$Alkyl, ein- oder mehrfach durch

Halogen substituiertes $C_1$-$C_3$-Alkyl, oder $C_1$-$C_3$-Alkoxy oder ein- oder mehrfach durch Halogen substituiertes $C_1$-$C_3$-Alkoxy oder $CH_2X$-$R_6$ ;
$R_6$ Wasserstoff, $C_1$-$C_3$-Alkyl, $C_3$-$C_4$-Alkenyl oder 2-Propinyl ;
$R_3$, $R_4$ und $R_5$ unabhängig voneinander Wasserstoff oder $C_1$-$C_3$-Alkyl ;
einschliesslich der Säureadditionssalze und Metallkomplexe.
3c) R unabhängig voneinander Halogen oder Methyl ;
m 0 bis 2 ;
n 0 oder 1 ;
X Sauerstoff ;
Y Schwefel ;

Z 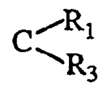 ;

Q N ;

A das Ringglied 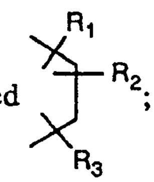 ;

$R_1$ und $R_2$ unabhängig voneinander Wasserstoff, Halogen, Hydroxy, $C_1$-$C_3$Alkyl oder $C_1$-$C_3$-Alkoxy oder ein- oder mehrfach durch Halogen substituiertes $C_1$-$C_3$-Alkoxy ;
$R_3$ Wasserstoff oder $C_1$-$C_2$-Alkyl ;
einschliesslich der Säureadditionssalze und Metallkomplexe.
4a) R unabhängig voneinander Halogen, $C_1$-$C_4$-Alkyl, $C_1$-$C_3$-Alkoxy, $C_1$-$C_3$-Haloalkyl, $C_1$-$C_3$-Haloalkoxy, $NO_2$ oder CN ;
m 1 bis 5 ;
n 0 bis 4 ;
X Sauerstoff oder Schwefel ;
Y Sauerstoff ;

Z 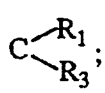 ;

Q N oder CH ;

A das Ringglied 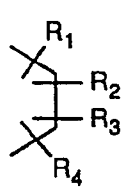 oder 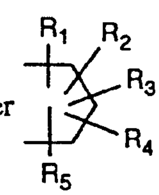 ;

$R_1$ und $R_2$ unabhängig voneinander Wasserstoff, Halogen, Hydroxy, $C_1$-$C_6$Alkyl, ein- oder mehrfach durch Halogen substituiertes $C_1$-$C_6$-Alkyl, oder $C_1$-$C_3$-Alkoxy oder ein- oder mehrfach durch Halogen substituiertes $C_1$-$C_3$-Alkoxy oder $CH_2X$-$R_6$ oder $C_3$-$C_6$-Cycloalkyl ;
$R_6$ Wasserstoff, $C_1$-$C_3$-Alkyl, $C_3$-$C_4$-Alkenyl, 2-Propinyl, 3-Halogen-2-propinyl, ein- oder mehrfach durch Halogen und/oder $C_1$-$C_2$-Alkoxy substituiertes $C_1$-$C_3$-Alkyl, durch $(R)_m$ substituiertes Phenyl oder durch $(R)_m$ substituiertes Benzyl ;
$R_3$, $R_4$ und $R_5$ unabhängig voneinander Wasserstoff oder $C_1$-$C_3$-Alkyl ;
einschliesslich der Säureadditionssalze und Metallkomplexe.
4b) R unabhängig voneinander Halogen, $C_1$-$C_4$-Alkyl, $C_1$-$C_3$-Alkoxy, $C_1$-$C_3$-Haloalkyl oder $C_1$-$C_3$-Haloalkoxy ;

m 1 bis 3 ;
n 0 bis 2 ;
X Sauerstoff oder Schwefel ;
Y Sauerstoff ;

Z 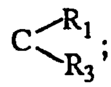 ;

Q N oder CH ;

A das Ringglied 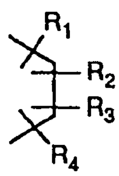 oder 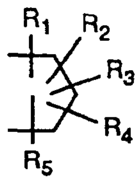 ;

$R_1$ und $R_2$ unabhängig voneinander Wasserstoff, Halogen, Hydroxy, $C_1$-$C_3$Alkyl, ein- oder mehrfach durch Halogen substituiertes $C_1$-$C_3$-Alkyl, oder $C_1$-$C_3$-Alkoxy oder ein- oder mehrfach durch Halogen substituiertes $C_1$-$C_3$-Alkoxy oder $CH_2X$-$R_6$ ;
$R_6$ Wasserstoff, $C_1$-$C_3$-Alkyl, ein- oder mehrfach durch Halogen und/oder $C_1$-$C_2$-Alkoxy substituiertes $C_1$-$C_3$-Alkyl ;
$R_3$, $R_4$ und $R_5$ unabhängig voneinander Wasserstoff oder $C_1$-$C_3$-Alkyl ;
einschliesslich der Säureadditionssalze und Metallkomplexe.
4c) R unabhängig voneinander Halogen, $C_1$-$C_2$-Alkyl, Methoxy, $CF_3$ oder $OCHF_2$ ;
m 1 bis 3 ;
n 0 bis 2 ;
X Sauerstoff oder Schwefel ;
Y Sauerstoff ;

Z 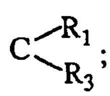 ;

Q N oder CH ;

A das Ringglied 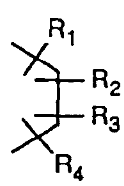 oder 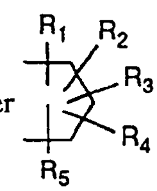 ;

$R_1$ und $R_2$ unabhängig voneinander Wasserstoff, Halogen, Hydroxy, $C_1$-$C_3$Alkyl, $CF_3$ oder $C_1$-$C_3$-Alkoxy oder ein- oder mehrfach durch Halogen substituiertes $C_1$-$C_3$-Alkoxy ;
$R_3$, $R_4$ und $R_5$ unabhängig voneinander Wasserstoff oder $C_1$-$C_3$-Alkyl ;
einschliesslich der Säureadditionssalze und Metallkomplexe.
4d) R unabhängig voneinander Halogen oder Methyl ;
m 1 bis 2 ;
n 0 oder 1 ;
X und Y Sauerstoff ;

Z 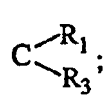 ;

Q N ;

A das Ringglied 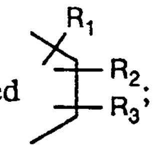

$R_1$ und $R_2$ unabhängig voneinander Wasserstoff, Halogen, Hydroxy, $C_1$-$C_3$Alkyl oder $C_1$-$C_3$-Alkoxy oder ein- oder mehrfach durch Halogen substituiertes $C_1$-$C_3$-Alkoxy ;
$R_3$ Wasserstoff oder $C_1$-$C_3$-Alkyl ;
einschliesslich der Säureadditionssalze und Metallkomplexe.

Die folgenden Verbindungen zeichnen sich durch besonders vorteilhaft pflanzenschützende Eigenschaften aus :

2-[4-(4-Chlorphenoxy)-2-chlorphenyl]-2-(1H-1,2,4-triazol-1-yl-methyl)-4-methyl-1,3-dithiolan ;
2-[4-(4-Bromphenoxy)-2-chlorphenyl]-2-(1H-1,2,4-triazol-1-yl-methyl)-thiazolidin ;
2-[4-(4-Chlorphenoxy)-2-chlorphenyl]-2-(1H-1,2,4-triazol-1-yl-methyl)-4-methyl-1,3-oxathiolan ;
2-[4-(4-Fluorphenoxy)-2-chlorphenyl]-2-(1H-1,2,4-triazol-1-yl-methyl)-1,3-dithiolan ;
2-[4-(4-Bromphenoxy)-2-chlorphenyl]-2-(1H-1,2,4-triazol-1-yl-methyl)-1,3-oxathiolan ;
2-[4-(4-Chlorphenoxy)-2-chlorphenyl]-2-(1H-1,2,4-triazol-1-yl-methyl)-1,3-oxathiolan ;
2-[4-(4-Chlorphenoxy)-2-chlorphenyl]-2-(1H-1,2,4-triazol-1-yl-methyl)-1,3-dithiolan ;
1-[(4-Brom-2-[4-(4-chlorphenoxy)-2-chlorphenyl]-tetrahydro-2-furanyl)-methyl]-1H-1,2,4-triazol ;
5-[4-(4-Chlorphenoxy)-2-chlorphenyl]-tetrahydro-5-(1H-1,2,4-triazol-1-ylmethyl-2-furyl-2,2,2-trifluorethyl ether.

Die Verbindungen der Formel I werden hergestellt, indem man umsetzt :

A) eine Verbindung der Formel II

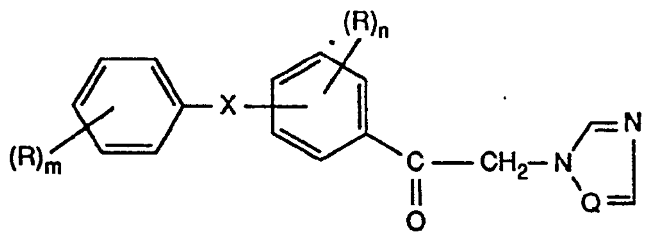

(II)

mit einer Verbindung der Formel III

H-Y-A-Z'-H (III),

worin Z' Schwefel oder N-$R_a$ darstellt und R, m, n, Q, Y und A die unter Formel I angegebenen Bedeutungen haben, in Gegenwart einer Säure in inerten organischen Lösungsmitteln bei den Rückflusstemperaturen der eingesetzten Lösungsmittel ; oder

B) eine Verbindung der Formel IV

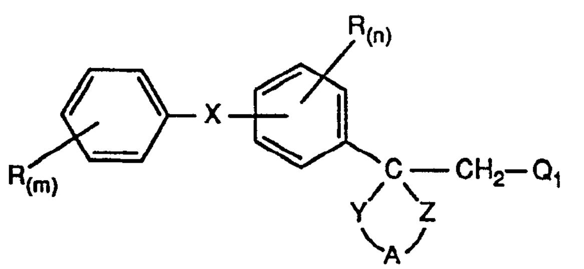

(IV)

mit einer Verbindung der Formel V

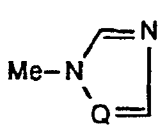

(V),

worin Me Wasserstoff oder ein Metallkation, vorzugsweise ein Alkalimetallatom, und $Q_1$ eine nucleophile Abgangsgruppe darstellen und X, Y, A, Q, R, m, n und Z die unter der Formel I angegebenen Bedeutungen besitzen, in relativ polaren inerten organischen Lösungsmitteln bei Temperaturen von 0° bis 220°C, bevorzugt 120°-170°C ; oder

C) eine Verbindung der Formel VI

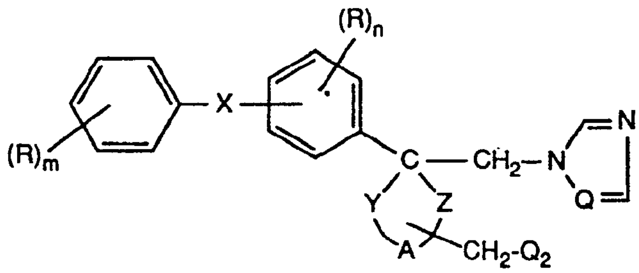

(VI)

mit einer Verbindung der Formel VII

$R_8$-$Q_3$ (VII),

worin $Q_2$ oder $Q_3$ eine gegebenenfalls in Salzform vorliegende Hydroxy- oder Mercaptogruppe darstellt, während der andere Rest ($Q_2$ oder $Q_3$) eine nucleophile Abgangsgruppe bedeutet, und R, X, Y, A, Z, Q, m und n die unter Formel I angegebenen Bedeutungen besitzen, mit der Massgabe, dass falls $R_1$ und $R_2$ den Rest $CH_2X$-$R_8$ darstellen, $R_8$ nicht Wasserstoff sein darf, wobei die Umsetzung im Falle, dass $Q_2$ und $Q_3$ Hydroxygruppen sind in Form einer Kondensation stattfindet ; oder

D) eine Verbindung der Formel VIII

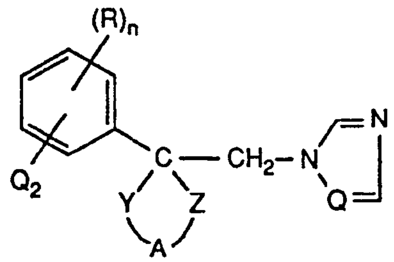

(VIII)

mit einer Verbindung der Formel IX

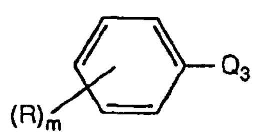

(IX)

worin $Q_2$ oder $Q_3$ eine gegebenenfalls in Salzform vorliegende Hydroxy- oder Mercaptogruppe darstellt, während der andere Rest ($Q_2$ oder $Q_3$) eine nucleophile Abgangsgruppe bedeutet, und R, X, Y, A, Z, Q, m und n die unter Formel I angegebenen Bedeutungen besitzen, wobei die Umsetzung im Falle, dass $Q_2$ und $Q_3$ Hydroxygruppen sind in Form einer Kondensation stattfindet ; oder

E) eine Verbindung der Formel X

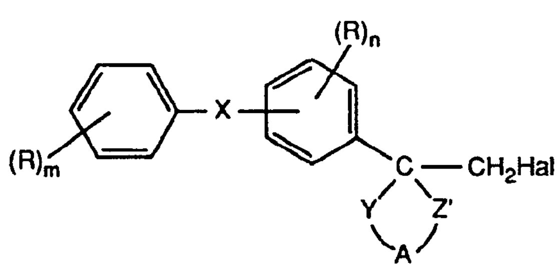

(X)

mit einer Verbindung der Formel XI

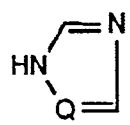

(XI),

worin R, X, Y, A, Q, m und n die unter Formel I angegebenen Bedeutungen besitzen, Z' den Rest $C(R_1)R_2$ darstellt und Hal Halogen, vorzugsweise Chlor, Brom oder Jod, bedeutet, in Gegenwart eines säurebindenden Mittels, wie z.B. einer Alkalibase, in einem inerten organischen lösungsmittel bei Temperaturen von 60° bis 160°C.

Bei der Verfahrensvariante (A) wird die Reaktion vorteilhafterweise in einem Ueberschuss von Verbindung III gegenüber Verbindung II durchgeführt. Die angewendete Säure stellt eine Lewis-Säure dar, wie z.B. Methansulfonsäure, Phenylsulfonsäure, p-Toluolsulfonsäure, Aluminiumchlorid, Zinkchlorid, Zinnchlorid usw., wobei p-Toluolsulfonsäure bevorzugt ist. Als geeignete inerte organische Lösungsmittel kommen z.B. in Frage : Kohlenwasserstoffe, wie Hexan, Cyclohexan, Heptan, Benzol, Toluol oder Xylol, ferner niedere Alkohole, wie z.B. die Butanole, sowie chlorierte Kohlenwasserstoffe, wie z.B. perchlorierte Methane oder Ethane. Darüber hinaus können auch Ether, wie z.B. Tetrahydrofuran oder 1,4-Dioxan, Anwendung finden. Auch Gemische von inerten Lösungsmitteln sind geeignet, wobei Mischungen aus Toluol und einem niederen Alkohol, vorzugsweise 1-Butanol, bevorzugt sind. Das während der Reaktion entstehende Wasser wird azeotropisch entfernt.

Diese Ketalisierungsreaktion kann analog zu bereits bekannten Ketalisierungs-Reaktionen durchgeführt werden, beispielsweise analog zur Herstellung von 2-Brommethyl-2,4-diphenyl- 1,3-dioxolan [Synthesis, 1974 (I), 23].

Bei der Ketalisierung werden beide Reaktionspartner mehrere Stunden zusammen mit einem Azeotrop-Bildner in einem der üblichen organischen Lösungsmittel unter Rückfluss erhitzt. Als Azeotrop-Bildner kommen z.B. Benzol, Toluol, Xylol, Chloroform oder Tetrachlorkohlenstoff in Frage, wobei zur Beschleunigung der Reaktion ein Zusatz einer starken Säure, wie z.B. p-Toluolsulfonsäure, vorteilhaft sein kann. Verwendbare organische Lösungsmittel sind in diesem Fall z.B. aromatische Kohlenwasserstoffe, wie Benzol, Toluol, Xylol usw., gesättigte Kohlenwasserstoffe, wie n-Hexan oder gesättigte halogenierte Kohlenwasserstoffe, wie z.B. 1,1,1-Trichlorethan.

Stellt in der Verfahrensvariante (B) in Formel V Me Wasserstoff dar, so wird das Verfahren in Gegenwart einer Base durchgeführt. Beispiele solcher Basen sind anorganische Basen wie die Oxide, Hydroxide, Hydride,Carbonate und Hydrogencarbonate von Alkali und Erdalkalimetallen, wie z.B. tert. Amine wie Triethylamin, Triethylendiamin, Piperidin, Pyridin, 4-Dimethylaminopyridin, 4-Pyrrolidylpyridin usw..

Metallkationen Me sind beispielsweise Alkalimetall-, z.B. Lithium-, Natrium- oder Kaliumkationen, oder Erdalkalimetall, z.B. Magnesium-, Calcium-, Strontium- oder Bariumkationen.

In den vorstehend beschriebenen Reaktionen können reaktionsinerte Lösungsmittel, wie z.B. N,N-Dimethylformamid, N,N-Dimethylacetamid, Hexamethylphosphortriamid, Dimethylsulfoxid, 4-Methyl-3-pentanon usw., verwendet werden. Auch Gemische mit anderen reaktionsinerten Lösungsmitteln, z.B. mit aromatischen Kohlenwasserstoffen, wie Benzol, Toluol, Xylol usw., können geeignet sein. In manchen Fällen kann es sich als vorteilhaft erweisen, zur Beschleunigung der Reaktionsgeschwindigkeit in Gegenwart einer Base zu arbeiten. Solche Basen sind z.B. Alkalimetallhydride oder Alkalimetallcarbonate.

In der Verfahrensvariante (B) stellt der Rest $Q_1$ eine nucleophile Abgangsgruppe dar. Als solche können beispielsweise folgende Verwendung finden : reaktionsfähige veresterte Hydroxygruppen, wie solche mit einer Halogenwasserstoffsäure, z.B. mit Fluor-, Chlor-, Brom- oder Jodwasserstoffsäure, oder mit einer Niederalkansulfonsäure, oder mit einer gegebenenfalls substituierten Benzol- oder Halogensulfonsäure ; beispielsweise mit Methan-, Ethan-, Benzol-, p-Toluol- oder Fluorsulfonsäure veresterte Hydroxygruppen.

Werden die Verbindungen der Formel I als Basen erhalten, lassen sie sich mit anorganischen oder organischen Säuren in entsprechende Salze oder mit vorzugsweise äquimolaren Mengen von Metallsalzen in Metallkomplexe der Formel I überführen. Umgekehrt lassen sich Salze der Formel I beispielsweise durch Reaktion mit Alkali(hydrogen)carbonat oder Alkalihydroxid in die freien Basen der Formel I überführen.

Die gemäss der Verfahrensvariante (A) hergestellten Verbindungen der Formel I stellen aufgrund der Ketalisierungsreaktion vorwiegend Gemische von Diastereomeren des resultierenden Ketals dar. Die Trennung dieser Diastereomerenpaare kann beispielsweise durch fraktionierte Kristallisation oder durch Chromatographie (Dünn-, Dickschicht-, Säulenchromatographie, Flüssigkeitshochdruckchromatographie usw.) erfolgen. Die beiden Isomeren zeigen unterschiedliche biologische Wirkungen. Im allgemeinen werden für praktische Zwecke die Diastereomerengemische verwendet. Ebenso wie die Diastereomerengemische stellen die durch Trennung isolierten Isomeren ein Gegenstand der vorliegenden Erfindung dar.

Bei den hier beschriebenen Herstellungsvarianten können die Zwischen- und Endprodukte aus dem Reaktionsmedium isoliert und, falls erwünscht, auf eine der allgemein üblichen Methoden gereinigt werden, z.B. durch Extraktion, Kristallisation, Chromatographie, Destillation usw.

Aus der Literatur sind bereits Phenylheterocyclyl-methylazol-Derivate bekannt. Solche Stoffe sind z.B. in der europäischen Patentanmeldung Nr. 243 983 und in dem australischen Patent Nr. 8 541 930 als Wirksubstanzen gegen schädliche Mikroorganismen, darunter auch phytopathogene Fungi, beschrieben. Diese Substanzen erfüllen jedoch nicht immer die in der Praxis an sie gestellten Forderungen.

Ueberraschenderweise wurde gefunden, dass Verbindungen der Formel I ein für praktische Bedürfnisse sehr günstiges Mikrobizid-Spektrum gegen phytopathogene Pilze und Bakterien aufweisen. Sie besitzen sehr vorteilhafte kurative, präventive und systemische Eigenschaften und lassen sich zum Schutz von Kulturpflanzen einsetzen. Mit den Wirkstoffen der Formel I können an Pflanzen oder an Pflanzenteilen (Früchte, Blüten, Laubwerk, Stengel, Knollen, Wurzeln) von unterschiedlichen Nutzkulturen die auftretenden Mikroorganismen eingedämmt oder vernichtet werden, wobei auch später zuwachsende Pflanzenteile von schädlichen Mikroorganismen verschont bleiben.

Die Wirkstoffe sind gegen die den folgenden Klassen angehörenden phytopathogenen Pilze wirksam : Ascomyceten (z.B. Venturia, Podosphaera, Erysiphe, Monilinia, Uncinula) ; Basidiomyceten (z.B. die Gattungen Hemileia, Rhizoctonia, Puccinia) ; Fungi imperfecti (z.B. Botrytis, Helmintosporium, Fusarium, Septoria, Cercospora und Alternaria). Ueberdies wirken die Verbindungen der Formel I systemisch. Sie können ferner als Beizmittel zur Behandlung von Saatgut (Früchte, Knollen, Körner) und Pflanzenstecklingen zum Schutz vor Pilzinfektionen sowie gegen im Erdboden auftretende phytopathogene Pilze eingesetzt werden. Die erfindungsgemässen Wirkstoffe zeichnen sich durch besonders gute Pflanzenverträglichkeit aus.

Die Erfindung betrifft somit auch mikrobizide Mittel sowie die Verwendung der Verbindungen der Formel I zur Bekämpfung phytopathogener Mikroorganismen, insbesondere pflanzenschädigender Pilze bzw. die präventive Verhütung eines Befalls an Pflanzen.

Darüber hinaus schliesst die vorliegende Erfindung auch die Herstellung agrochemischer Mittel ein, die gekennzeichnet ist durch das innige Vermischen der Aktivsubstanz mit einem oder mehreren hierin beschriebenen Substanzen bzw. Substanzgruppen. Die Erfindung bezieht sich ferner auf ein Verfahren zur Behandlung von Pflanzen, das sich durch Applikation der Verbindungen der Formel I bzw. der neuen Mittel auszeichnet.

Als Zielkulturen für die hierin offenbarten Indikationsgebiete gelten im Rahmen dieser Erfindung z.B. folgende Pflanzenarten : Getreide : (Weizen, Gerste, Roggen, Hafer, Reis, Sorghum und Verwandte) ; Rüben : (Zucker- und Futterrüben) ; Kern-, Stein- und Beerenobst : (Aepfel, Birnen, Pflaumen, Pfirsiche, Mandeln, Kirschen, Erd-, Him- und Brombeeren) ; Hülsenfrüchte : (Bohnen, Linsen, Erbsen, Soja) ; Oelkulturen : (Raps, Senf, Mohn, Oliven, Sonnenblumen, Kokos, Rizinus, Kakao, Erdnüsse) ; Gurkengewächse : (Kürbis, Gurken, Melonen) ; Fasergewächse : (Baumwolle, Flachs, Hanf, Jute) ; Citrusfrüchte : (Orangen, Zitronen, Pampelmusen, Mandarinen) ; Gemüsesorten : (Spinat, Kopfsalat, Spargel, Kohlarten, Möhren, Zwiebeln, Tomaten, Kartoffeln, Paprika) ; Lorbeergewächse : (Avocado, Cinnamonum, Kampfer) oder Pflanzen wie Mais, Tabak, Nüsse, Kaffee, Zuckerrohr, Tee, Weinreben, Hopfen, Bananen- und Naturkautschukgewächse sowie Zierpflanzen (Blumen, Sträucher, Laubbäume und Nadelbäume wie Koniferen). Diese Aufzählung stellt keine Limitierung dar.

Wirkstoffe der Formel I werden üblicherweise in Form von Zusammensetzungen verwendet und können gleichzeitig oder nacheinander mit weiteren Wirkstoffen auf die zu behandelnde Fläche oder Pflanze gegeben werden. Diese weiteren Wirkstoffe können sowohl Düngemittel, Spurenelement-Vermittler oder andere das Pflanzenwachstum beeinflussende Präparate sein. Dazu zählen aber auch selektive Herbizide, Insektizide, Fungizide, Bakterizide, Nematizide, Molluskizide oder Gemische mehrerer dieser Präparate zusammen mit gegebenenfalls weiteren in der Formulierungstechnik üblichen Trägerstoffen, Tensiden oder anderen applikationsfördernden Zusätzen.

Bei allen Formulierungen können die geeigneten Träger und Zusätze fest oder flüssig sein, wobei sie den in der Formulierungstechnik zweckdienlichen Stoffen entsprechen, wie z.B. natürlichen oder regenerierten mineralischen Stoffen, Lösungs-, Dispergier-, Netz-, Haft-, Verdickungs-, Binde- oder Düngemitteln.

Ein bevorzugtes Verfahren zum Aufbringen eines Wirkstoffes der Formel I bzw. eines agrochemischen Mittels, das mindestens einen dieser Wirkstoffe enthält, ist das Aufbringen auf das Blattwerk (Blattapplikation). Anzahl der Applikationen und Aufwandmenge richten sich dabei nach dem Befallsdruck des entsprechenden Erregers (Pilzspecies). Die Wirkstoffe der Formel I können aber auch über den Erdboden durch das Wurzelwerk in die Pflanze gelangen (systemische Wirkung), indem man den Standort der Pflanze mit einer flüssigen Zubereitung tränkt oder die Substanzen in fester Form in den Boden einbringt z.B. in Form von Granulat (Bodenapplikation). Die Verbindungen der Formel I können aber auch auf Samenkörner aufgebracht werden (Coating), indem man die Körner entweder in einer flüssigen Zubereitung des Wirkstoffs tränkt oder sie mit einer festen Zubereitung beschichtet. Darüber hinaus sind in besonderen Fällen weitere Applikationsarten möglich, so z.B.

die gezielte Behandlung der Pflanzenstengel oder der Knospen.

Die Verbindungen der Formel I werden dabei in unveränderter Form oder vorzugsweise zusammen mit den in der Formulierungstechnik üblichen Hilfsmittel eingesetzt und werden daher z.B. zu Emulsionskonzentraten, streichfähigen Pasten, direkt versprühbaren oder verdünnbaren Lösungen, verdünnten Emulsionen, Spritzpulvern, löslichen Pulvern, Stäubemitteln, Granulaten, durch Verkapselungen in z.B. polymeren Stoffen in bekannter Weise verarbeitet. Die Anwendungsverfahren wie Versprühen, Vernebeln, Verstäuben, Verstreuen, Bestreichen oder Giessen werden gleich wie die Art der Mittel den angestrebten Zielen und den gegebenen Verhältnissen entsprechend gewählt. Günstige Aufwandmengen liegen im allgemeinen bei 50 g bis 5 kg Aktivsubstanz (AS) je ha ; bevorzugt 100 g bis 2 kg AS/ha, insbesondere bei 100 g bis 600 g AS/ha.

Die Formulierungen, d.h. die den Wirkstoff der Formel I und gegebenenfalls einen festen oder flüssigen Zusatzstoff enthaltenden Mittel, Zubereitungen oder Zusammensetzungen werden in bekannter Weise hergestellt, z.B. durch inniges Vermischen und/oder Vermahlen der Wirkstoffe mit Streckmitteln, wie z.B. mit Lösungsmitteln, festen Trägerstoffen, und gegebenfalls oberflächenaktiven Verbindungen (Tensiden).

Als Lösungsmittel können in Frage kommen : Aromatische Kohlenwasserstoffe, bevorzugt die Fraktionen $C_8$ bis $C_{12}$, wie z.B. Xylolgemische oder substituierte Naphthaline, Phthalsäureester wie Dibutyl- oder Dioctylphthalat, aliphatische Kohlenwasserstoffe wie Cyclohexan oder Paraffine, Alkohole und Glykole sowie deren Ether und Ester, wie Ethanol, Ethylenglykol, Ethylenglykolmonomethyl- oder Ethylether, Ketone wie Cyclohexanon, stark polare Lösungsmittel wie N-Methyl-2-pyrrolidon, Dimethylsulfoxid oder Dimethylformamid, sowie gegebenenfalls epoxydierte Pflanzenöle wie epoxydiertes Kokosnussöl oder Sojaöl ; oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen wie z.B. Bimsstein, Ziegelbruch, Sepiolit oder Bentonit, als nicht sorptive Trägermaterialien z.B. Calcit oder Sand in Frage. Darüber hinaus kann eine Vielzahl von vorgranulierten Materialien anorganischer oder organischer Natur wie insbesondere Dolomit oder zerkleinerte Pflanzenrückstände verwendet werden.

Als oberflächenaktive Verbindungen kommen je nach Art des zu formulierenden Wirkstoffes der Formel I nichtionogene, kation- und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen, als auch wasserlösliche synthetische oberflächenaktive Verbindungen sein.

Als Seifen seien die Alkali-, Erdalkali- oder gegebenenfalls substituierten Aminoniumsalze von höheren Fettsäuren ($C_{10}$-$C_{22}$), wie z.B. die Na- oder K-Salze der Oel- oder Stearinsäure, oder von natürlichen Fettsäuregemischen, die z.B. aus Kokosnuss- oder Talgöl gewonnen werden können, genannt.

Als synthetische Tenside können insbesondere Fettalkoholsulfonate, Fettalkoholsulfate, sulfonierte Benzimidazolderivate oder Alkylsulfonate Verwendung finden. Die Fettalkoholsulfonate oder -sulfate liegen in der Regel als Alkali-, Erdalkali- oder gegebenenfalls substituierte Ammoniumsalze vor und weisen einen Alkalirest mit 8 bis 22 C-Atomen auf.

Als nicht-ionische Tenside kommen in erster Linie Polyglykoletherderivate von aliphatischen oder cycloaliphatischen Alkoholen, gesättigten oder ungesättigten Fettsäuren und Alkylphenolen in Frage, die 3 bis 30 Glykolethergruppen und 8 bis 20 Kohlenstoffatome im (aliphatischen) Kohlenwasserstoffrest und 6 bis 18 Kohlenstoffatome im Alkylrest der Alkylphenole enthalten können.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

Die agrochemischen Zubereitungen enthalten in der Regel 0,1 bis 99 Gew.-%, insbesondere 0,1 bis 95 Gew.-%, Wirkstoff der Formel I, 99,9 bis 1 Gew.-%, insbesondere 99,8 bis 5 Gew.-%, eines festen oder flüssigen Zusatzstoffes und 0 bis 25 Gew.-%, insbesondere 0,1 bis 25 Gew.-%, eines Tensides.

Die nachfolgenden Beispiele dienen zur näheren Erläuterung der Erfindung, ohne dieselbe einzuschränken.

1. Herstellungsbeispiele

1.1. Herstellung von 2-[4-(4-Chlorphenoxy)-2-methylphenyl]-2-(1H-1,2,4-triazol-1-yl-methyl)-thiazolidin

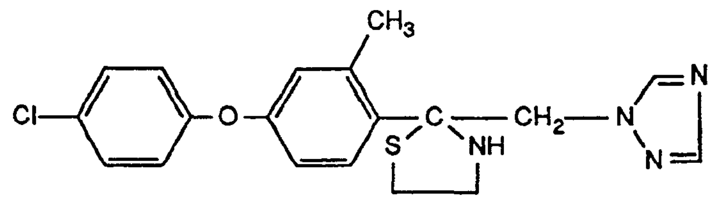

16,4 g 2-[4-(4-Chlorphenoxy)-2-methylphenyl]-2-(1H-1,2,4-triazol-1-yl)-ethan-2-on, 11,4 g β-Mercaptoethylamin-Hydrochlorid und 10,1 g Triethylamin werden in je 100 ml Toluol und n-Butanol am Wasserabscheider zum Sieden erhitzt. Nach 10 Stunden am Rückfluss wird auf Raumtemperatur abgekühlt und die weissgelbe Suspension 2 mal mit je 300 ml Wasser gewaschen. Die organische Phase wird am Rotationsverdampfer eingeengt. Der feste Rückstand wird zur Reinigung mittels Ethylacetat/Diethylether ( 1 :1 ) über eine Kieselgelsäule chromatographiert. Die Titelverbindung wird in Form weisser Kristalle erhalten Smp. 112-113°C.

1.2. Herstellung von 2-[4-(4-Bromphenoxy)-2-chlorphenyl]-2-(1H-1,2,4-triazol-1-yl-methyl)-1,3-dithiolan

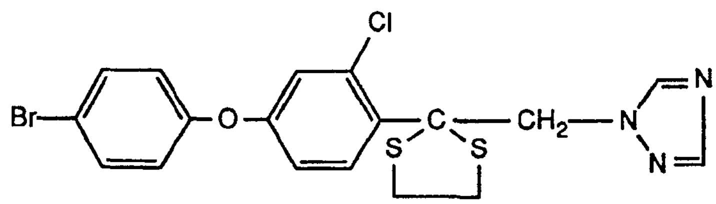

5,9 g 2-[4-(4-Bromphenoxy)-2-chlorphenyl]-2-(1H-1,2,4-triazol-1-yl)-ethan-2-on und 5,7 g p-Toluolsulfonsäure-monohydrat werden in je 50 ml Toluol und n-Butanol 1,5 Stunden am Wasserabscheider gekocht. Dann wird auf ~80°C abgekühlt, 2,1 g 1,2-Ethandithiol zugegeben und wieder zum Sieden erhitzt. Nach 18 Stunden am Wasserabscheider werden nochmals 5,7 g p-Toluolsulfonsäure-monohydrat und 2,1 g 1,2-Ethandithiol zugegeben. Nach weiteren 70 Stunden Rückfluss am Wasserabscheider wird auf Raumtemperatur abgekühlt und die gelbe Suspension filtriert. Der feste Rückstand wird gut mit Toluol nachgewaschen, anschliessend in 80 ml Chloroform und 50 ml Wasser aufgenommen und mit Kaliumkarbonat alkalisch gestellt. Die organische Phase wird abgetrennt, 2 mal mit je 100 ml Wasser gewaschen, über Natriumsulfat getrocknet, filtriert und am Rotationsverdampfer eingeengt. Der feste Rückstand wird zur Reinigung mittels Methylenchlorid/Diethylether (1 :1) über eine Kieselgelsäule chromatographiert. Die Titelverbindung wird in Form weisser Kristalle erhalten. Smp. 133-135°C.

1.3. Herstellung von 2-[4-(4-Chlorphenoxy)-2-chlorphenyl]-2-(1H-1,2,4-triazol-1-yl-methyl)-1,3-oxathiolan

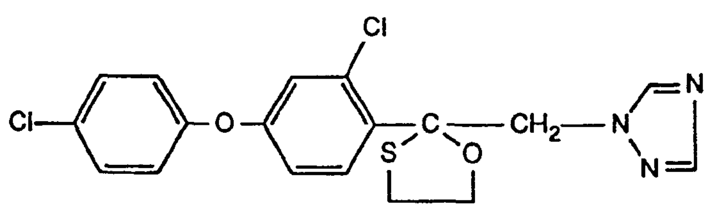

16,5 g 2-[4-(4-Chlorphenoxy)-2-chlorphenyl]-2-(1H-1,2,4-triazol-1-yl)-ethan-2-on und 18,0 g p-Toluolsulfonsäure-monohydrat werden in 40 ml n-Butanol und 160 ml Toluol am Wasserabscheider zum Sieden erhitzt. Nach 4 Stunden Rückfluss wird auf ca. 60°C abgekühlt, 5,6 g 2-Mercaptoethanol zugegeben und weitere 16 Stunden am Wasserabscheider gekocht. Dann werden nochmals 40 ml n-Butanol und 5,6 g 2-Mercapto-ethanol zugegeben und weitere 22 Stunden am Rückfluss gekocht. Nach dem Abkühlen auf Raumtemperatur wird die feine Suspension abfiltriert, der feste Rückstand mit Toluol nachgewaschen, das Filtrat mit Kaliumkarbonat

neutralisiert, 2 mal mit je 150 ml Wasser gewaschen, über Natriumsulfat getrocknet, filtriert und am Vakuum das Lösungsmittel abgedampft. Der gelbe, flüssige Rückstand wird zur Reinigung mittels Methylenchlorid/Diethylether (1 :1) über eine Kieselgelsäule chromatographier. Das ölige Produkt wird mit wenig Diethylether/Petrolether (1 :2) kristallisiert. Smp. der gelblichen Kristalle 89,5-91°C.

Tabelle 1:

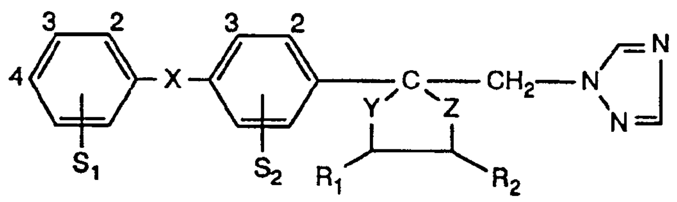

| No. | $S_1$ | $S_2$ | X | $R_1$ | $R_2$ | Y | Z | physikalische Daten |
|---|---|---|---|---|---|---|---|---|
| 1.1 | 4-Cl | 2-$CH_3$ | O | H | H | S | NH | Smp. 112-113°C |
| 1.2 | 4-Cl | 3-Cl | O | H | H | O | S | |
| 1.3 | 2,4-$Cl_2$ | 2-Cl | O | H | H | S | NH | |
| 1.4 | 4-Cl | 2-Cl | S | H | H | S | NH | |
| 1.5 | H | 2-Cl | O | H | H | S | S | |
| 1.6 | 4-F | 2-Cl | O | H | H | S | NH | Smp. 99-101°C |
| 1.7 | 4-Cl | 2-Cl | S | $CH_3$ | H | S | NH | |
| 1.8 | 4-Cl | 2-Cl | O | H | $CH_3$ | S | NH | |
| 1.9 | 4-F | 2-Cl | O | $CH_3$ | H | S | NH | |
| 1.10 | 4-Br | 2-Cl | O | H | H | S | S | Smp. 133-135°C |
| 1.11 | 4-Cl | 2-Br | O | $CH_3$ | H | S | NH | |
| 1.12 | 4-Cl | 2-Cl | O | $CH_3$ | H | S | NH | |
| 1.13 | 4-Br | 2-$CH_3$ | O | H | H | S | NH | |
| 1.14 | 4-Cl | 2-Cl | O | H | H | S | N-$C_2H_5$ | |
| 1.15 | 4-F | 2-Cl | O | H | H | O | S | Smp. 105-107°C |
| 1.16 | 4-Cl | 2-Cl | O | $CH_3$ | H | S | S | $n_D^{41}$: 1.6207 |
| 1.17 | 4-Cl | 2-Cl | O | H | $C_2H_5$ | S | S | |
| 1.18 | 4-$NO_2$ | 2-Cl | O | H | H | S | NH | |
| 1.19 | 4-$CH_3$ | 2-$CH_3$ | O | H | H | O | S | |
| 1.20 | 4-Cl | 2-Cl | O | $C_2H_5$ | H | S | NH | |
| 1.21 | 3-Cl | 3-Cl | O | H | H | S | NH | $n_D^{23}$: 1,6323 |
| 1.22 | 4-$NO_2$ | 2-$CH_3$ | O | H | H | S | S | |
| 1.23 | 2,4-$Cl_2$ | 2-Br | O | H | H | O | S | |
| 1.24 | 4-F | 2-Cl | S | H | H | S | S | |
| 1.25 | 4-Cl | 2-Cl | S | H | H | O | S | |
| 1.26 | 4-Cl | 2-Cl | O | H | H | S | NH | Smp. 100-102°C |
| 1.27 | 4-F | 2-F | O | H | H | S | NH | |
| 1.28 | 4-Cl | 2,3$(CH_3)_2$ | O | H | H | S | S | |
| 1.29 | 4-F | 2-F | O | H | H | O | S | |
| 1.30 | 4-Cl | 2,3$(CH_3)_2$ | O | H | H | O | S | |
| 1.31 | 4-Br | 2-Br | O | H | H | S | NH | |
| 1.32 | 4-CN | 2-Cl | O | H | H | S | NH | |
| 1.33 | 4-Br | 2-Br | O | H | H | O | S | |
| 1.34 | 4-Br | 2-Cl | O | H | H | S | NH | Smp. 114-115°C |
| 1.35 | 4-Cl | 2-$OCHF_2$ | O | H | H | S | NH | |
| 1.36 | 4-Br | 2-Br | O | H | H | S | S | |
| 1.37 | 4-Cl | 2-Cl | O | H | H | S | N-$CH_3$ | |
| 1.38 | 4-Br | 2-Cl | O | $CH_3$ | H | S | NH | |
| 1.39 | 4-Cl | 2-$OCH_3$ | O | H | H | S | NH | |

Tabelle 1: (Fortsetzung)

| No. | $S_1$ | $S_2$ | X | $R_1$ | $R_2$ | Y | Z | physikalische Daten |
|---|---|---|---|---|---|---|---|---|
| 1.40 | 4-Cl | 2-Br | O | H | H | S | NH | Smp. 110-111°C |
| 1.41 | 4-Br | 2-Cl | O | H | $CH_3$ | S | NH | |
| 1.42 | 4-$OCHF_2$ | 2-Cl | O | H | H | O | S | |
| 1.43 | 2,4,5$Cl_3$ | 2-Cl | O | H | H | S | NH | |
| 1.44 | 4-F | 2-$CH_3$ | O | H | H | O | S | Smp. 77-79°C |
| 1.45 | 4-Cl | 2-Cl | O | $C_2H_5$ | H | O | S | |
| 1.46 | 4-Cl | 2-Cl | O | $CH_3$ | H | O | S | |
| 1.47 | 4-Cl | 2-Cl | O | H | $CH_3$ | O | S | |
| 1.48 | 4-F | 2-Cl | O | H | H | S | S | Smp. 115-116,5°C |
| 1.49 | 2-Cl | 4-Cl | O | $CH_3$ | $CH_3$ | S | S | |
| 1.50 | 2-Cl | 4-Cl | O | $CH_3$ | $CH_3$ | O | S | |
| 1.51 | 4-Br | 2-Cl | O | H | H | O | S | Smp. 105-107°C |
| 1.52 | 4-Br | 2-Cl | O | H | H | S | N-$C_3H_7$-n | |
| 1.53 | 4-Cl | 2-Cl | O | H | H | O | S | Smp. 89,5-91°C |
| 1.54 | 4-Br | 2-Cl | O | $CH_3$ | H | S | S | |
| 1.55 | 4-Cl | 2-Cl | O | H | H | S | S | Smp. 128-129°C |
| 1.56 | 4-Cl | 2-Cl | O | $CH_2OCH_3$ | H | O | S | |
| 1.57 | 4-Cl | 2-Cl | O | H | $CH_2OCH_3$ | O | S | |
| 1.58 | 4-Cl | 2-Cl | O | $CH_2OCH_3$ | H | S | S | |
| 1.59 | 4-Cl | 2-Cl | O | 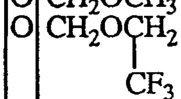 | H | S | S | |
| 1.60 | 4-Cl | 2-Cl | O | 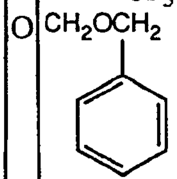 | H | O | S | |
| 1.61 | 4-Cl | 2-Cl | O | 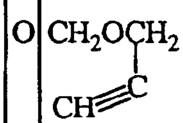 | H | S | S | |
| 1.62 | 4-Cl | 2-Cl | O | $CH_3$ | H | S | S | $n_D^{48}$: 1.6118 (cis-Isomeres) |
| 1.63 | 4-Cl | 2-Cl | O | H | $CH_2CH_3$ | O | S | |
| 1.64 | 4-Cl | 2-Cl | O | $C_3H_7$-n | H | S | S | |
| 1.65 | 4-Cl | 2-$CH_3$ | O | H | $CH_3$ | S | S | $n_D^{41}$: 1.6243 (Isomeren-Gemisch) |
| 1.66 | 4-Cl | 2-$CH_3$ | O | H | $CH_3$ | S | S | $n_D^{42}$: 1.6081 (Cis-Isomeres) |
| 1.67 | 4-Cl | 2-$CH_3$ | O | H | $CH_3$ | S | S | $n_D^{50}$: 1.6213 (Trans-Isomeres) |

Tabelle 2:

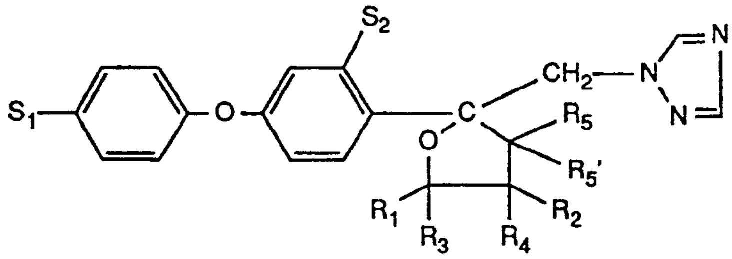

| No. | $S_1$ | $S_2$ | $R_1$ | $R_2$ | $R_3$ | $R_4$ | $R_5$ | $R_{5'}$ | physikalische Daten |
|---|---|---|---|---|---|---|---|---|---|
| 2.1 | Cl | Cl | $CH_3$ | H | H | H | H | H | |
| 2.2 | Cl | Cl | H | H | H | H | H | H | Smp. 87-90°C |
| 2.3 | Br | Cl | H | H | H | H | H | H | Smp. 92-94°C |
| 2.4 | Cl | Br | $CH_3$ | H | H | H | H | H | |
| 2.5 | Cl | Cl | H | $CH_3$ | H | H | H | H | |
| 2.6 | F | F | H | H | H | H | H | H | |
| 2.7 | Cl | Cl | $CH_3$ | H | $CH_3$ | H | H | H | |
| 2.8 | Cl | Cl | H | H | H | H | $CH_3$ | H | |
| 2.9 | Cl | Cl | H | H | H | H | $CH_3$ | $CH_3$ | |
| 2.10 | Br | Cl | $CH_3$ | $CH_3$ | $CH_3$ | $CH_3$ | H | H | |
| 2.11 | Cl | Cl | $C_2H_5$ | H | H | H | H | H | |
| 2.12 | Cl | Cl | $CH_3$ | $CH_3$ | $CH_3$ | $CH_3$ | H | H | |
| 2.13 | Cl | Cl | OH | H | H | H | H | H | |
| 2.14 | Cl | Cl | $OCH_3$ | $CH_3$ | H | H | H | H | |
| 2.15 | Cl | Cl | $OC_2H_5$ | H | H | H | H | H | |
| 2.16 | F | Cl | $OCH_3$ | H | H | H | H | H | |
| 2.17 | Br | Cl | $OCH_3$ | H | H | H | H | H | |
| 2.18 | Cl | Cl | H | Br | H | H | H | H | |
| 2.19 | Cl | Cl | H | Cl | H | H | H | H | |
| 2.20 | Cl | Cl | $OCH_2CF_3$ | H | H | H | H | H | Smp. 101-103°C |
| 2.21 | Br | Cl | H | Br | H | H | H | H | |
| 2.22 | Br | Br | H | Br | H | H | H | H | |
| 2.23 | F | F | H | Br | H | H | H | H | |
| 2.24 | F | Cl | H | Br | H | H | H | H | |
| 2.25 | Cl | Cl | H | OH | H | H | H | H | |
| 2.26 | Cl | Cl | H | $OCH_3$ | H | H | H | H | |
| 2.27 | Cl | Cl | H | Br | H | H | $CH_3$ | H | |
| 2.28 | Br | Cl | $OCH_2CF_3$ | H | H | H | H | H | |
| 2.29 | F | Cl | $OCH_2CF_3$ | H | H | H | H | H | Smp. 105-108°C |
| 2.30 | Cl | Br | $OCH_2CF_3$ | H | H | H | H | H | |

Tabelle 3:

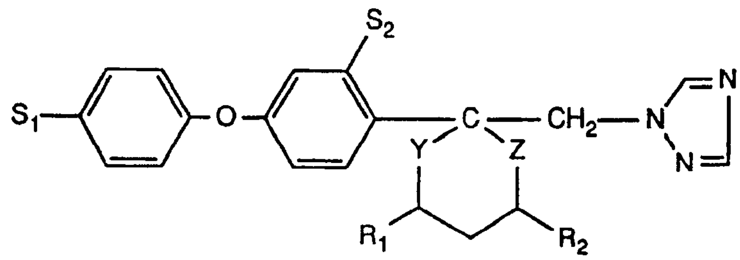

| No. | $S_1$ | $S_2$ | Y | Z | $R_1$ | $R_2$ | physikalische Daten |
|---|---|---|---|---|---|---|---|
| 3.1 | Cl | Cl | S | S | H | H | Smp. 115-118°C |
| 3.2 | Br | Cl | S | S | H | H | Smp. 122-124°C |
| 3.3 | Cl | Cl | S | S | $CH_3$ | H | |
| 3.4 | Cl | Cl | S | NH | H | H | Smp. 89-91°C |
| 3.5 | Cl | Cl | O | S | $CH_3$ | $CH_3$ | |
| 3.6 | Br | Cl | O | S | H | H | |
| 3.7 | Cl | Cl | O | S | H | H | Smp. 95-96°C |
| 3.8 | Br | Cl | S | NH | H | H | |
| 3.9 | Cl | Cl | O | S | $CH_3$ | H | |
| 3.10 | Cl | Cl | O | S | H | $CH_3$ | |
| 3.11 | F | Cl | S | S | H | H | |
| 3.12 | Cl | $CH_3$ | S | S | H | H | |
| 3.13 | Cl | $CH_3$ | O | S | H | H | |
| 3.14 | Cl | Br | S | S | H | H | |
| 3.15 | Cl | Cl | O | $CH_2$ | H | H | |
| 3.16 | F | F | O | S | H | H | |

2. Formulierungsbeispiele für flüssige Wirkstoffe der Formel I (% = Gewichtsprozent)

| 2.1. Emulsions-Konzentrate | a) | b) | c) |
|---|---|---|---|
| Wirkstoff aus den Tabellen 1-3 | 25 % | 40 % | 50 % |
| Ca-Dodecylbenzolsulfonat | 5 % | 8 % | 6 % |
| Ricinusöl-polyethylenglykolether (36 Mol Ethylenoxid) | 5 % | - | - |
| Tributylphenoyl-polyethylen-glykolether (30 Mol Ethylenoxid) | - | 12 % | 4 % |
| Cyclohexanon | - | 15 % | 20 % |
| Xylolgemisch | 65 % | 25 % | 20 % |

Aus solchen Konzentraten können durch Verdünnen mit Wasser Emulsionen jeder gewünschten Konzentration hergestellt werden.

| 2.2. Lösungen | a) | b) | c) | d) |
|---|---|---|---|---|
| Wirkstoff aus den Tabellen 1-3 | 80 % | 10 % | 5 % | 95 % |
| Ethylenglykol-monomethyl-ether | 20 % | - | - | - |
| Polyethylenglykol MG 400 | - | 70 % | - | - |
| N-Methyl-2-pyrrolidon | - | 20 % | - | - |
| Epoxydiertes Kokosnussöl | - | - | 1 % | 5 % |
| Benzin (Siedegrenzen 160-190°C) | - | - | 94 % | - |

(MG = Molekulargewicht)

Die Lösungen sind zur Anwendung in Form kleinster Tropfen geeignet.

| 2.3. Granulate | a) | b) |
|---|---|---|
| Wirkstoff aus den Tabellen 1-3 | 5 % | 10 % |
| Kaolin | 94 % | - |
| Hochdisperse Kieselsäure | 1 % | - |
| Attapulgit | - | 90 % |

Der Wirkstoff wird in Methylenchlorid gelöst, auf den Träger aufgesprüht und das Lösungsmittel anschliessend im Vakuum abgedampft.

| 2.4. Stäubemittel | a) | b) |
|---|---|---|
| Wirkstoff aus den Tabellen 1-3 | 2 % | 5 % |
| Hochdisperse Kieselsäure | 1 % | 5 % |
| Talkum | 97 % | - |
| Kaolin | - | 90 % |

Durch inniges Vermischen auf Trägerstoffe mit dem Wirkstoff erhält man gebrauchsfertige Stäubemittel.

Formulierungsbeispiele für feste Wirkstoffe der Formel I (% = Gewichtsprozent)

| 2.5. Spritzpulver | a) | b) | c) |
|---|---|---|---|
| Wirkstoff aus den Tabellen 1-3 | 25 % | 50 % | 75 % |
| Na-Ligninsulfonat | 5 % | 5 % | - |
| Na-Laurylsulfat | 3 % | - | 5 % |
| Na-Diisobutylnaphthalinsulfonat | - | 6 % | 10 % |
| Octylphenolpolyethylenglykolether (7-8 Mol Ethylenoxid) | - | 2 % | - |
| Hochdisperse Kieselsäure | 5 % | 10 % | 10 % |
| Kaolin | 62 % | 27 % | - |

Der Wirkstoff wird mit den Zusatzstoffen gut vermischt und in einer geeigneten Mühle pot vermahlen. Man

erhält Spritzpulver, die sich mit Wasser zu Suspensionen jeder gewünschten Konzentration verdünnen lassen.

| 2.6. Emulsions-Konzentrat | |
|---|---|
| Wirkstoff aus den Tabellen 1-3 | 10 % |
| Octylphenolpolyethylenglykolether (4-5 Mol Ethylenoxid) | 5 % |
| Ca-Dodecylbenzolsulfonat | 1 % |
| Ricinusölpolyglykolether (35 Mol Ethylenoxid) | 4 % |
| Cyclohexanon | 30 % |
| Xylolgemisch | 50 % |

Aus diesem Konzentrat können durch Verdünnen mit Wasser Emulsionen jeder gewünschten Konzentration hergestellt werden.

| 2.7. Stäubemittel | a) | b) |
|---|---|---|
| Wirkstoff aus den Tabellen 1-3 | 5 % | 8 % |
| Talkum | 95 % | - |
| Kaolin | - | 92 % |

Man erhält anwendungsfertige Stäubemittel, indem der Wirkstoff mit dem Träger vermischt und auf einer geeigneten Mühle vermahlen wird.

| 2.8. Extruder-Granulat | |
|---|---|
| Wirkstoff aus den Tabellen 1-3 | 10 % |
| Na-Ligninsulfonat | 2 % |
| Carboxymethylcellulose | 1 % |
| Kaolin | 87 % |

Der Wirkstoff wird mit den Zusatzstoffen vermischt, vermahlen und mit Wasser angefeuchtet. Dieses Gemisch und extrudiert und anschliessend im Luftstrom getrocknet.

| 2.9. Umhüllungs-Granulat | |
|---|---|
| Wirkstoff aus den Tabellen 1-3 | 3 % |
| Polyethlyenglykol (MG 200) | 3 % |
| Kaolin | 94 % |
| (MG = Molekulargewicht) | |

Der fein gemahlene Wirkstoff wird in einem Mischer auf das mit Polyethylenglykol angefeuchtete Kaolin gleichmässig aufgetragen. Auf diese Weise erhält man staubfreie Umhüllungs-Granulate.

2.10. Suspensions-Konzentrat

| | |
|---|---|
| Wirkstoff aus den Tabellen 1-3 | 40 % |
| Ethlyenglykol | 10 % |
| Nonylphenolpolyethylenglykolether (15 Mol Ethylenoxid) | 6 % |
| N-Ligninsulfonat | 10 % |
| Carboxymethylcellulose | 1 % |
| 37%ige wässrige Formaldehyd-Lösung | 0,2 % |
| Silikonöl in Form einer 75%igen wässrigen Emulsion Wasser | 0,8 % |
| | 32 % |

Der fein gemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

3. Biologische Beispiele :

Beispiel 3.1 : Wirkung gegen Puccinia graminis auf Weizen

a) Residual-preotekive Wirkung

Weizenpflanzen werden 6 Tage nach der Aussaat mit einer aus Spritzpulver des Wirkstoffes hergestellten Spritzbrühe (0,02 % Aktivsubstanz) besprüht. Nach 24 Stunden werden die behandelten Pflanzen mit einer Uredosporensuspension des Pilzes infiziert. Nach einer Inkubation während 48 Stunden bei 95-100 % relativer Luftfeuchtigkeit und ca. 20°C werden die infizierten Pflanzen in einem Gewächshaus bei ca. 22°C aufgestellt. Die Beurteilung der Rostpustelentwicklung erfolgt 12 Tage nach der Infektion.

b) Systemische Wirkung

Zu Weizenpflanzen wird 5 Tage nach der Aussaat eine aus Spritzpulver des Wirkstoffes hergestellte Spritzbrühe gegossen (0,006 % Aktivsubstanz bezogen auf das Bodenvolumen). Nach 48 Stunden werden die behandelten Pflanzen mit einer Uredosporensuspension des Pilzes infiziert. Nach einer Inkubation während 48 Stunden bei 95-100 % relativer Luftfeuchtigkeit und ca. 20°C werden die infizierten Pflanzen in einem Gewächshaus bei ca. 22°C aufgestellt. Die Beurteilung der Rostpustelentwicklung erfolgt 12 Tage nach der Infektion.

Verbindungen aus den Tabellen 1 bis 3 zeigen gegen den Puccinia-Pilz eine gute Wirkung. Unter anderem hemmten die Verbindungen Nr. 1.15, 1.16, 1.34, 1.44, 1.53 und 1.55 bei residual-protektiver Behandlung den Pilzbefall auf 0 bis 10 %. Unbehandelte aber infizierte Kontrollpflanzen wiesen dagegen einen Puccinia-Befall von 100 % auf.

Beispiel 3.2 : Wirkung gegen Cercospora arachidicola auf Erdnusspflanzen

10-15 cm hohe Erdnusspflanzen werden mit einer aus Spritzpulver der Wirksubstanz hergestellten Spritzbrühe (0,02 % Aktivsubstanz) besprüht und 48 Stunden später mit einer Konidiensuspension des Pilzes infiziert. Die infizierten Pflanzen werden während 72 Stunden bei ca. 21°C und hoher Luftfeuchtigkeit inkubiert und anschliessend bis zum Auftreten der typischen Blattflecken in einem Gewächshaus aufgestellt. Die Beurteilung der fungiziden Wirkung erfolgt 12 Tage nach der Infektion basierend auf Anzahl und Grösse der auftretenden Flecken.

Im Vergleich zu unbehandelten, aber infizierten Kontrollpflanzen (Anzahl und Grösse der Flecken = 100 %), zeigen Erdnusspflanzen, die mit Wirkstoffen aus den Tabellen 1 bis 3 behandelt werden, einen stark reduzierten Cercospora-Befall. So verhinderten die Verbindungen 1.6, 1.15, 1.16, 1.34, 1.44, 1.48, 1.51, 1.53, 1.55 und 2.18 in obigem Versuch das Auftreten von Flecken fast vollständig (0 bis 5 %).

Beispiel 3.3 : Wirkung gegen Erysiphae graminis auf Gerste

a) Residual-protektive Wirkung

Ca. 8 cm hohe Gerstenpflanzen werden mit einer aus Spritzpulver des Wirkstoffes hergestellten Spritzbrühe (0,02 % Aktivsubstanz) besprüht. Nach 3-4 Stunden werden die behandelten Pflanzen mit Konidien des Pilzes bestäubt. Die infizierten Gerstenpflanzen werden in einem Gewächshaus bei ca. 22°C aufgestellt und der Pilzbefall nach 10 Tagen beurteilt.

b) Systemische Wirkung

Zu ca. 8 cm Gerstenpflanzen wird eine aus Spritzpulver des Wirkstoffes hergestellte Spritzbrühe gegossen (0,006 % Aktivsubstanz bezogen auf das Erdvolumen). Es wird dabei darauf geachtet, dass die Spritzbrühe nicht mit den oberirdischen Pflanzenteilen in Berührung kommt. Nach 48 Stunden werden die behandelten Pflanzen mit Konidien des Pilzes bestäubt. Die infizierten Gerstenpflanzen werden in einem Gewächshaus bei ca. 22°C aufgestellt und der Pilzbefall nach 10 Tagen beurteilt.

Verbindungen der Formel I zeigen eine gute residual-protektive Wirkung gegen Erysiphe-Pilze. Unter anderen Verbindungen aus den Tabellen 1 bis 3 hemmten die Verbindungen Nr. 1.15, 1.16, 1.34, 1.44, 1.46, 1.48, 1.50 1.51, 1.53, 1.55, 2.18, 2.20 und 3.1den Pilzbefall auf Gerste auf weniger als 5 %. Unbehandelte, aber infizierte Kontrollpflanzen zeigten einen Erysiphe-Befall von 100 %.

Beispiel 3.4 : Residual-protektive Wirkung gegen Venturia inaequalis auf Apfeltrieben

Apfelstecklinge mit 10-20 cm langen Frischtrieben werden mit einer aus Spritzpulver des Wirkstoffes hergestellten Spritzbrühe (0,02 % Aktivsubstanz) besprüht. Nach 24 Stunden werden die behandelten Pflanzen mit einer Konidiensuspension des Pilzes infiziert. Die Pflanzen werden dann während 5 Tage bei 90-100 % relativer Luftfeuchtigkeit inkubiert und während 10 weiteren Tagen in einem Gewächshaus bei 20-24°C aufgestellt. Der Schorfbefall wird 15 Tage nach der Infektion beurteilt. Die Verbindungen Nr. 1.15, 1.26, 1.34, 1.44, 1.48, 1.51, 1.53 und 1.55 hemmten den Krankheitsbefall auf weniger als 10 %. Unbehandelte aber infizierte Triebe zeigten einen 100%-igen Venturia-Befall.

Beispiel 3.5 : Wirkung gegen Botrytis cinerea an Apfelfrüchten

Künstlich verletzte Aepfel werden behandelt, indem eine aus Spritzpulver der Wirksubstanz hergestellte Spritzbrühe (0,02 % Aktivsubstanz) auf die Verletzungsstellen aufgetropft wird. Die behandelten Früchte werden anschliessend mit einer Sporensuspension von Botryris cinerea inokuliert und während einer Woche bei einer hohen Luftfeuchtigkeit und ca. 20°C inkubiert.

Bei der Auswertung werden die angefaulten Verletzungsstellen gezählt und daraus die fungizide Wirkung der Testsubstanz abgeleitet. Verbindungen aus Tabelle 1 waren gut wirksam gegen Botrytis-Befall von Apfelfrüchten. Unter anderen hemmten die Verbindungen Nr. 1.26, 1.34, 1.40 und 1.53 den Pilzbefall im Vergleich zu unbehandelten Kontrollfrüchten (mit 100 % Befall) fast vollständig (Krankheitsbefall 0-5 %).

## Patentansprüche

1. Verbindungen der Formel I

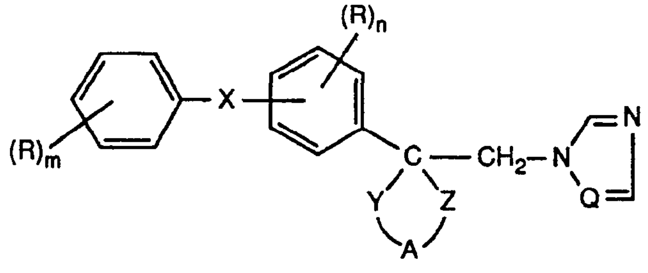

(I),

in welcher bedeuten :

R unabhängig voneinander Halogen, $C_1$-$C_4$-Alkyl, $C_1$-$C_3$-Alkoxy, $C_1$-$C_3$-Haloalkyl, $C_1$-$C_3$-Haloalkoxy, $NO_2$ oder CN ;
m 0 bis 5 ;
n 0 bis 4 ;
X und Y unabhängig voneinander Sauerstoff oder Schwefel ;

Z Schwefel, N-$R_a$ oder 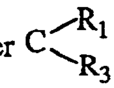 ;

$R_a$ Wasserstoff oder $C_1$-$C_6$-Alkyl ;
Q N oder CH ;

A das Ringglied 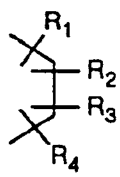 oder 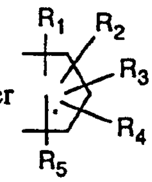 ;

$R_1$ und $R_2$ unabhängig voneinander Wasserstoff, Halogen, Hydroxy, $C_1$-$C_6$Alkyl, ein- oder mehrfach durch Halogen substituiertes $C_1$-$C_6$-Alkyl, oder $C_1$-$C_3$-Alkoxy oder ein- oder mehrfach durch Halogen substituiertes $C_1$-$C_3$-Alkoxy oder $CH_2X$-$R_6$ oder $C_3$-$C_6$-Cycloalkyl ;
$R_6$ Wasserstoff, $C_1$-$C_3$-Alkyl, $C_3$-$C_4$-Alkenyl, 2-Propinyl, 3-Halogen-2-propinyl, ein- oder mehrfach durch Halogen und/oder $C_1$-$C_2$-Alkoxy substituiertes $C_1$-$C_3$-Alkyl, durch $(R)_m$ substituiertes Phenyl oder durch $(R)_m$ substituiertes Benzyl ;
$R_3$, $R_4$ und $R_5$ unabhängig voneinander Wasserstoff, $C_1$-$C_3$-Alkyl ;
mit der Massgabe, dass falls Z Schwefel oder den Rest N-$R_a$ darstellt $n \geq 1$ sein muss und falls Y Sauerstoff, Z den Rest $C(R_1)R_3$ und X Sauerstoff darstellen $m \geq 1$ sein muss ; einschliesslich der Säureadditionssalze und Metallkomplexe der Verbindungen der Formel I.

**2.** Verbindungen gemäss Anspruch 1 der Formel I, in welcher bedeuten :
R unabhängig voneinander Halogen, $C_1$-$C_4$-Alkyl, $C_1$-$C_3$-Alkoxy, $C_1$-$C_3$-Haloalkyl, $C_1$-$C_3$-Haloalkoxy, $NO_2$ oder CN ;
m 0 bis 5 ;
n 1-4 ;
X und Y unabhängig voneinander Sauerstoff oder Schwefel ;
Z N-$R_a$ ;
$R_a$ Wasserstoff oder $C_1$-$C_6$-Alkyl ;
Q N oder CH ;

A das Ringglied 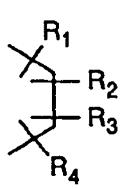 oder 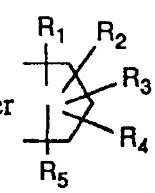 ;

$R_1$ und $R_2$ unabhängig voneinander Wasserstoff, $C_1$-$C_6$Alkyl, ein- oder mehrfach durch Halogen substituiertes $C_1$-$C_6$-Alkyl, oder $C_1$-$C_3$-Alkoxy oder $CH_2X$-$R_6$ oder $C_3$-$C_6$-Cycloalkyl ;
$R_6$ Wasserstoff, $C_1$-$C_3$-Alkyl, $C_3$-$C_4$-Alkenyl, 2-Propinyl, 3-Halogen-2-propinyl, ein- oder mehrfach durch Halogen und/oder $C_1$-$C_2$-Alkoxy substituiertes $C_1$-$C_3$-Alkyl, durch $(R)_m$ substituiertes Phenyl oder durch (R)m substituiertes Benzyl ;
$R_3$, $R_4$ und $R_5$ unabhängig voneinander Wasserstoff oder $C_1$-$C_3$-Alkyl ;
einschliesslich der Säureadditionssalze und Metallkomplexe.

**3.** Verbindungen gemäss Anspruch 2 der Formel I, in welcher bedeuten :
R unabhängig voneinander Halogen, $C_1$-$C_4$-Alkyl, Methoxy, $C_1$-$C_3$-Haloalkyl, $C_1$-$C_3$-Haloalkoxy ;

m 0-3 ;
n 0-2 ;
X Sauerstoff oder Schwefel ;
Y Schwefel ;
Z N-$R_a$ ;
$R_a$ Wasserstoff oder $C_1$-$C_6$-Alkyl ;
Q N oder CH ;

A das Ringglied 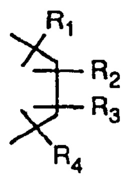 oder 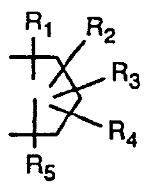 ;

$R_1$ und $R_2$ unabhängig voneinander Wasserstoff, $C_1$-$C_6$Alkyl, ein- oder mehrfach durch Halogen substituiertes $C_1$-$C_6$-Alkyl, oder $CH_2X$-$R_6$ ;
$R_6$ Wasserstoff, $C_1$-$C_3$-Alkyl, $C_3$-$C_4$-Alkenyl, 2-Propinyl, 3-Halogen-2-propinyl, ein- oder mehrfach durch Halogen und/oder $C_1$-$C_2$-Alkoxy substituiertes $C_1$-$C_3$-Alkyl, oder durch (R)m substituiertes Benzyl ;
$R_3$, $R_4$ und $R_5$ unabhängig voneinander Wasserstoff oder $C_1$-$C_3$-Alkyl ;
einschliesslich der Säureadditionssalze und Metallkomplexe.

4. Verbindungen gemäss Anspruch 2 der Formel 1, in welcher bedeuten :
R unabhängig voneinander Halogen, $C_1$-$C_2$-Alkyl, $CF_3$, $OCHF_2$ ;
m 0-2 ;
n 0-2 ;
X Sauerstoff oder Schwefel ;
Y Schwefel ;
Z N-$R_a$ ;
$R_a$ Wasserstoff oder $C_1$-$C_6$-Alkyl ;
Q N oder CH ;

A das Ringglied 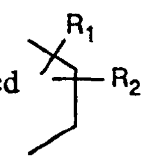 oder 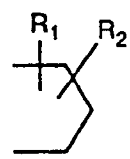 ;

$R_1$ und $R_2$ unabhängig voneinander Wasserstoff, $C_1$-$C_3$Alkyl, ein- oder mehrfach durch Halogen substituiertes $C_1$-$C_3$-Alkyl, oder $CH_2X$-$R_6$ ;
$R_6$ Wasserstoff, $C_1$-$C_3$-Alkyl, $C_3$-$C_4$-Alkenyl, 2-Propinyl oder 3-Halogen-2-propinyl ;
einschliesslich der Säureadditionssalze und Metallkomplexe.

5. Verbindungen gemäss Anspruch 2 der Formel I, in welcher bedeuten :
R unabhängig voneinander Halogen oder Methyl ;
m 0-2 ;
n 1 ;
X Sauerstoff ;
Y Schwefel ;
Z N-$R_a$ ;
$R_a$ Wasserstoff ;
Q N ;

A das Ringglied 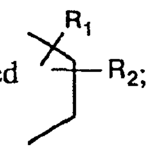 ;

$R_1$ und $R_2$ unabhängig voneinander Wasserstoff, $C_1$-$C_3$-Alkyl ; oder $CH_2O$-$R_6$ ;
$R_6$ Wasserstoff oder $C_1$-$C_3$-Alkyl ;
einschliesslich der Säureadditionssalze und Metallkomplexe.

6. Verbindungen gemäss Anspruch 1 der Formel I, in welcher bedeuten :
R unabhängig voneinander Halogen, $C_1$-$C_4$-Alkyl, $C_1$-$C_3$-Alkoxy, $C_1$-$C_3$-Haloalkyl, $C_1$-$C_3$-Haloalkoxy, $NO_2$ oder CN ;
m 0 bis 5 ;
n 1-4 ;
X und Y unabhängig voneinander Sauerstoff oder Schwefel ;
Z Schwefel ;
Q N oder CH ;

A das Ringglied 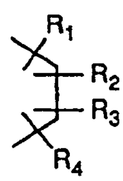 oder 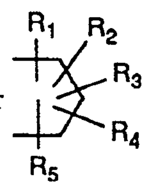 ;

$R_1$ und $R_2$ unabhängig voneinander Wasserstoff, $C_1$-$C_6$Alkyl, ein- oder mehrfach durch Halogen substituiertes $C_1$-$C_6$-Alkyl, oder $C_1$-$C_3$-Alkoxy oder $CH_2X$-$R_6$ oder $C_3$-$C_6$-Cycloalkyl ;
$R_6$ Wasserstoff, $C_1$-$C_3$-Alkyl, $C_3$-$C_4$-Alkenyl, 2-Propinyl, 3-Halogen-2-propinyl, ein- oder mehrfach durch Halogen und/oder $C_1$-$C_2$-Alkoxy substituiertes $C_1$-$C_3$-Alkyl, durch $(R)_m$ substituiertes Phenyl oder durch $(R)_m$ substituiertes Benzyl ;
$R_3$, $R_4$ und $R_5$ unabhängig voneinander Wasserstoff oder $C_1$-$C_3$-Alkyl ;
einschliesslich der Säureadditionssalze und Metallkomplexe.

7. Verbindungen gemäss Anspruch 6 der Formel 1, in welcher bedeuten :
R unabhängig voneinander Halogen, $C_1$-$C_4$-Alkyl, $C_1$-$C_3$-Alkoxy, $C_1$-$C_3$-Haloalkyl oder $C_1$-$C_3$-Haloalkoxy ;
m 0-3 ;
n 1-2 ;
X und Y unabhängig voneinander Sauerstoff oder Schwefel ;
Z Schwefel ;
Q N oder CH ;

A das Ringglied 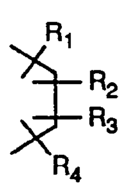 oder 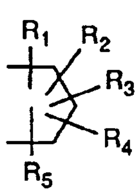 ;

$R_1$ und $R_2$ unabhängig voneinander Wasserstoff, $C_1$-$C_6$Alkyl, ein- oder mehrfach durch Halogen substituiertes $C_1$-$C_6$-Alkyl, oder $C_1$-$C_3$-Alkoxy oder $CH_2X$-$R_6$ ;
$R_6$ Wasserstoff, $C_1$-$C_3$-Alkyl, $C_3$-$C_4$-Alkenyl, 2-Propinyl, 3-Halogen-2-propinyl, ein- oder mehrfach durch Halogen und/oder $C_1$-$C_2$-Alkoxy substituiertes $C_1$-$C_3$-Alkyl, oder durch (R)m substituiertes Phenyl oder durch (R)m substituiertes Benzyl ;
$R_3$, $R_4$ und $R_5$ unabhängig voneinander Wasserstoff oder $C_1$-$C_3$-Alkyl ;
einschliesslich der Säureadditionssalze und Metallkomplexe.

8. Verbindungen gemäss Anspruch 6 der Formel I, in welcher bedeuten :

R unabhängig voneinander Halogen, $C_1$-$C_2$-Alkyl, Methoxy, $CF_3$ oder $OCHF_2$ ;
m 0-2 ;
n 1 ;
X und Y unabhängig voneinander Sauerstoff oder Schwefel ;
Z Schwefel ;
Q N oder CH ;

A das Ringglied 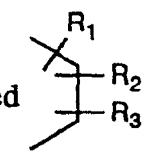 oder 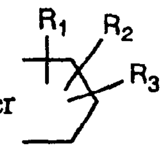 ;

$R_1$ und $R_2$ unabhängig voneinander Wasserstoff, $C_1$-$C_3$Alkyl, ein- oder mehrfach durch Halogen substituiertes $C_1$-$C_3$-Alkyl, oder $CH_2X$-$R_6$ ;
$R_6$ Wasserstoff, $C_1$-$C_3$-Alkyl, $C_3$-$C_4$-Alkenyl, 2-Propinyl oder 3-Halogen-2-propinyl ;
$R_3$ Wasserstoff oder $C_1$-$C_3$-Alkyl ;
einschliesslich der Säureadditionssalze und Metallkomplexe.

**9.** Verbindungen gemäss Anspruch 6 der Formel I, in welcher bedeuten :
R unabhängig voneinander Halogen oder Methyl ;
m 0 oder 1 ;
n 1 ;
X Sauerstoff ;
Y Sauerstoff oder Schwefel ;
Z Schwefel ;
Q N ;

A das Ringglied 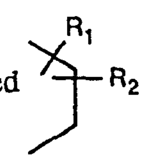 oder 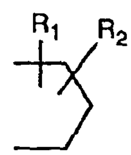 ;

$R_1$ und $R_2$ unabhängig voneinander Wasserstoff, $C_1$-$C_3$-Alkyl oder $CH_2O$-$R_6$ ;
$R_6$ Wasserstoff oder $C_1$-$C_3$-Alkyl ;
einschliesslich der Säureadditionssalze und Metallkomplexe.

**10.** Verbindungen gemäss Anspruch 1 der Formel I, in welcher bedeuten :
R unabhängig voneinander Halogen, $C_1$-$C_4$-Alkyl, $C_1$-$C_3$-Alkoxy, $C_1$-$C_3$-Haloalkyl, $C_1$-$C_3$-Haloalkoxy, $NO_2$ oder CN ;
m 0 bis 5 ;
n 0 bis 4 ;
X Sauerstoff oder Schwefel ;
Y Schwefel ;

Z 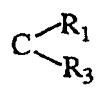 ;

Q N oder CH ;

A das Ringglied 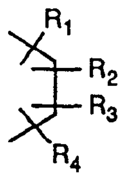 oder 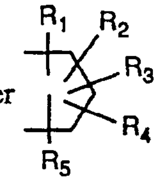 ;

$R_1$ und $R_2$ unabhängig voneinander Wasserstoff, Halogen, Hydroxy, $C_1$-$C_6$Alkyl, ein- oder mehrfach durch Halogen substituiertes $C_1$-$C_6$-Alkyl, oder $C_1$-$C_3$-Alkoxy oder ein- oder mehrfach durch Halogen substituiertes $C_1$-$C_3$-Alkoxy oder $CH_2X$-$R_6$ oder $C_3$-$C_6$-Cycloalkyl ;
$R_6$ Wasserstoff, $C_1$-$C_3$-Alkyl, $C_3$-$C_4$-Alkenyl, 2-Propinyl, 3-Halogen-2-propinyl, ein- oder mehrfach durch Halogen und/oder $C_1$-$C_2$-Alkoxy substituiertes $C_1$-$C_3$-Alkyl, durch $(R)_m$ substituiertes Phenyl oder durch $(R)_m$ substituiertes Benzyl ;
$R_3$, $R_4$ und $R_5$ unabhängig voneinander Wasserstoff oder $C_1$-$C_3$-Alkyl ;
einschliesslich der Säureadditionssalze und Metallkomplexe.

**11.** Verbindungen gemäss Anspruch 10 der Formel I, in welcher bedeuten :
R unabhängig voneinander Halogen, $C_1$-$C_4$-Alkyl, Methoxy, $CF_3$ oder $OCHF_2$ ;
m 0 bis 3 ;
n 0 bis 2 ;
X Sauerstoff oder Schwefel ;
Y Schwefel ;

Z 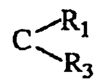 ;

Q N oder CH ;

A das Ringglied 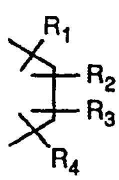 oder 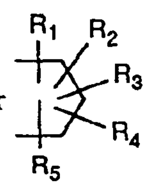 ;

$R_1$ und $R_2$ unabhängig voneinander Wasserstoff, Halogen, Hydroxy, $C_1$-$C_3$Alkyl, ein- oder mehrfach durch Halõgen substituiertes $C_1$-$C_3$-Alkyl, oder $C_1$-$C_3$-Alkoxy oder ein- oder mehrfach durch Halogen substituiertes $C_1$-$C_3$-Alkoxy oder $CH_2X$-$R_6$ ;
$R_6$ Wasserstoff, $C_1$-$C_3$-Alkyl, $C_3$-$C_4$-Alkenyl oder 2-Propinyl ;
$R_3$, $R_4$ und $R_5$ unabhängig voneinander Wasserstoff oder $C_1$-$C_3$-Alkyl ;
einschliesslich der Säureadditionssalze und Metallkomplexe.

**12.** Verbindungen gemäss Anspruch 10 der Formel I, in welcher bedeuten :
R unabhängig voneinander Halogen oder Methyl ;
m 0 bis 2 ;
n 0 oder 1 ;
X Sauerstoff ;
Y Schwefel ;

Z 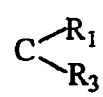 ;

Q N ;

A das Ringglied 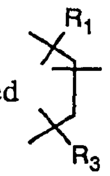 ;

$R_1$ und $R_2$ unabhängig voneinander Wasserstoff, Halogen, Hydroxy, $C_1$-$C_3$Alkyl oder $C_1$-$C_3$-Alkoxy oder ein- oder mehrfach durch Halogen substituiertes $C_1$-$C_3$-Alkoxy ;
$R_3$ Wasserstoff oder $C_1$-$C_2$-Alkyl ;
einschliesslich der Säureadditionssalze und Metallkomplexe.

13. Verbindungen gemäss Anspruch 1 der Formel I, in welcher bedeuten :
R unabhängig voneinander Halogen, $C_1$-$C_4$-Alkyl, $C_1$-$C_3$-Alkoxy, $C_1$-$C_3$-Haloalkyl, $C_1$-$C_3$-Haloalkoxy, $NO_2$ oder CN ;
m 1 bis 5 ;
n 0 bis 4 ;
X Sauerstoff oder Schwefel ;
Y Sauerstoff ;

Z 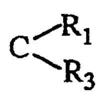 ;

Q N oder CH ;

A das Ringglied 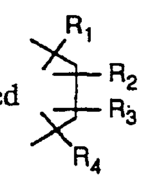 oder 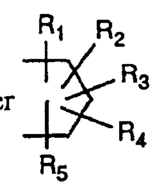 ;

$R_1$ und $R_2$ unabhängig voneinander Wasserstoff, Halogen, Hydroxy, $C_1$-$C_6$Alkyl, ein- oder mehrfach durch Halogen substituiertes $C_1$-$C_6$-Alkyl, oder $C_1$-$C_3$-Alkoxy oder ein- oder mehrfach durch Halogen substituiertes $C_1$-$C_3$-Alkoxy oder $CH_2X$-$R_6$ oder $C_3$-$C_6$-Cycloalkyl ;
$R_6$ Wasserstoff, $C_1$-$C_3$-Alkyl, $C_3$-$C_4$-Alkenyl, 2-Propinyl, 3-Halogen-2-propinyl, ein- oder mehrfach durch Halogen und/oder $C_1$-$C_2$-Alkoxy substituiertes $C_1$-$C_3$-Alkyl, durch (R)m substituiertes Phenyl oder durch (R)m substituiertes Benzyl ;
$R_3$, $R_4$ und $R_5$ unabhängig voneinander Wasserstoff oder $C_1$-$C_3$-Alkyl ;
einschliesslich der Säureadditionssalze und Metallkomplexe.

14. Verbindungen gemäss Anspruch 13 der Formel I, in welcher bedeuten :
R unabhängig voneinander Halogen, $C_1$-$C_4$-Alkyl, $C_1$-$C_3$-Alkoxy, $C_1$-$C_3$-Haloalkyl oder $C_1$-$C_3$-Haloalkoxy ;
m 1 bis 3 ;
n 0 bis 2 ;
X Sauerstoff oder Schwefel ;
Y Sauerstoff ;

Z 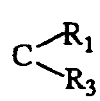 ;

Q N oder CH ;

A das Ringglied 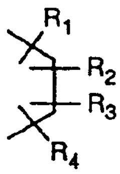 oder 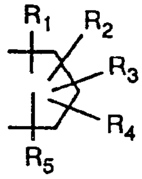 ;

$R_1$ und $R_2$ unabhängig voneinander Wasserstoff, Halogen, Hydroxy, $C_1$-$C_3$Alkyl, ein- oder mehrfach durch Halogen substituiertes $C_1$-$C_3$-Alkyl, oder $C_1$-$C_3$-Alkoxy oder ein- oder mehrfach durch Halogen substituiertes $C_1$-$C_3$-Alkoxy oder $CH_2X$-$R_8$ ;
$R_8$ Wasserstoff, $C_1$-$C_3$-Alkyl, ein- oder mehrfach durch Halogen und/oder $C_1$-$C_2$-Alkoxy substituiertes $C_1$-$C_3$-Alkyl ;
$R_3$, $R_4$ und $R_5$ unabhängig voneinander Wasserstoff oder $C_1$-$C_3$-Alkyl ;
einschliesslich der Säureadditionssalze und Metallkomplexe.

**15.** Verbindungen gemäss Anspruch 13 der Formel I, in welcher bedeuten :
R unabhängig voneinander Halogen, $C_1$-$C_2$-Alkyl, Methoxy, CF oder $OCHF_2$ ;
m 1 bis 3 ;
n 0 bis 2 ;
X Sauerstoff oder Schwefel ;
Y Sauerstoff ;

Z 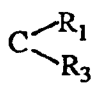 ;

Q N oder CH ;

A das Ringglied 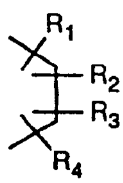 oder 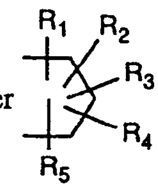 ;

$R_1$ und $R_2$ unabhängig voneinander Wasserstoff, Halogen, Hydroxy, $C_1$-$C_3$Alkyl, $CF_3$ oder $C_1$-$C_3$-Alkoxy oder ein- oder mehrfach durch Halogen substituiertes $C_1$-$C_3$-Alkoxy ;
$R_3$, $R_4$ und $R_6$ unabhängig voneinander Wasserstoff oder $C_1$-$C_3$-Alkyl ;
einschliesslich der Säureadditionssalze und Metallkomplexe.

**16.** Verbindungen gemäss Anspruch 13 der Formel I, in welcher bedeuten :
R unabhängig voneinander Halogen oder Methyl ;
m 1 bis 2 ;
n 0 oder 1 ;
X und Y Sauerstoff ;

Z 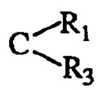 ;

Q N ;

A das Ringglied 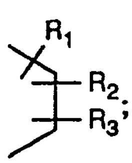 ;

$R_1$ und $R_2$ unabhängig voneinander Wasserstoff, Halogen, Hydroxy, $C_1$-$C_3$Alkyl oder $C_1$-$C_3$-Alkoxy oder ein- oder mehrfach durch Halogen substituiertes $C_1$-$C_3$-Alkoxy ;
$R_3$ Wasserstoff oder $C_1$-$C_3$-Alkyl ;
einschliesslich der Säureadditionssalze und Metallkomplexe.

17. Eine Verbindung aus der Gruppe :
2-[4-(4-Chlorphenoxy)-2-chlorphenyl]-2-(1H-1,2,4-triazol-1-yl-methyl)-4-methyl-1,3-dithiolan ;
2-[4-(4-Bromphenoxy)-2-chlorphenyl]-2-(1H-1,2,4-triazol-1-yl-methyl)-thiazolidin ;
2-[4-(4-Chlorphenoxy)-2-chlorphenyl]-2-(1H-1,2,4-triazol-1-yl-methyl)-4-methyl-1,3-oxathiolan ;
2-[4-(4-Fluorphenoxy)-2-chlorphenyl]-2-(1H-1,2,4-triazol-1-yl-methyl)-1,3-dithiolan ;
2-[4-(4-Bromphenoxy)-2-chlorphenyl]-2-(1H-1,2,4-triazol-1-yl-methyl)-1,3-oxathiolan ;
2-[4-(4-Chlorphenoxy)-2-chlorphenyl]-2-(1H-1,2,4-triazol-1-yl-methyl)-1,3-oxathiolan ;
2-[4-(4-Chlorphenoxy)-2-chlorphenyl]-2-(1H-1,2,4-triazol-1-yl-methyl)-1,3-dithiolan ;
1-[(4-Brom-2-[4-(4-chlorphenoxy)-2-chlorphenyl]-tetrahydro-2-furanyl)-methyl]- 1H-1,2,4-triazol ;
5-[4-(4-Chlorphenoxy)-2-chlorphenyl]-tetrahydro-5-(1H-1,2,4-triazol-1-ylmethyl-2-furyl-2,2,2-trifluorethyl ether.

18. Verfahren zur Herstellung von Verbindungen der Formel I in Anspruch 1, dadurch gekennzeichnet, dass man umsetzt :
A) eine Verbindung der Formel II

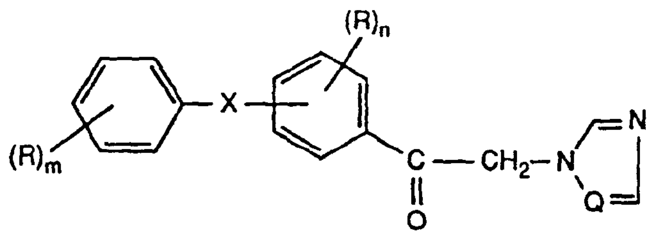

(II)

mit einer Verbindung der Formel III

H-Y-A-Z'-H (III),

worin Z'Schwefel oder N-$R_a$ darstellt und R, m, n, Q, Y und A die unter Formel I angegebenen Bedeutungen haben, in Gegenwart einer Säure in inerten organischen Lösungsmitteln bei den Rückflusstemperaturen der eingesetzten Lösungsmittel ; oder
B) eine Verbindung der Formel IV

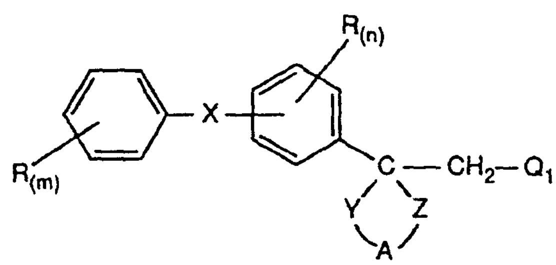

(IV)

mit einer Verbindung der Formel V

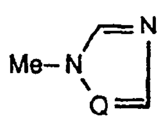

(V),

worin Me Wasserstoff oder ein Metallkation, vorzugsweise ein Alkalimetallatom, und $Q_1$ eine nucleophile Abgangsgruppe darstellen und X, Y, A, Q, R, m, n und Z die unter der Formel I angegebenen Bedeutungen besitzen, in relativ polaren inerten organischen Lösungsmitteln bei Temperaturen von 0° bis 220°C ; oder

C) eine Verbindung der Formel VI

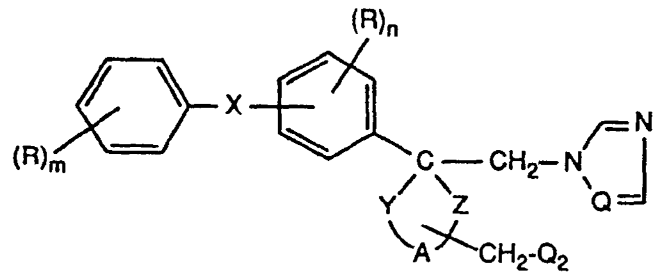

(VI)

mit einer Verbindung der Formel VII

$R_6$-$Q_3$ (VII),

worin $Q_2$ oder $Q_3$ eine gegebenenfalls in Salzform vorliegende Hydroxy- oder Mercaptogruppe darstellt, während der andere Rest ($Q_2$ oder $Q_3$) eine nucleophile Abgangsgruppe bedeutet, und R, X, Y, A, Z, Q, m und n die unter Formel I angegebenen Bedeutungen besitzen, mit der Massgabe, dass falls $R_1$ und $R_2$ den Rest $CH_2X$-$R_6$ darstellen, $R_6$ nicht Wasserstoff sein darf ; oder

D) eine verbindung der Formel VIII

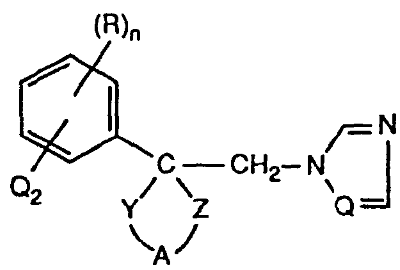

(VIII)

mit einer Verbindung der Formel IX

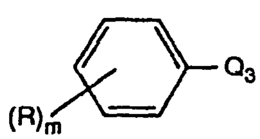

(IX)

worin $Q_2$ oder $Q_3$ eine gegebenenfalls in Salzform vorliegende Hydroxy- oder Mercaptogruppe darstellt, während der andere Rest ($Q_2$ oder $Q_3$) eine nucleophile Abgangsgruppe bedeutet, und R, X, Y, A, Z, Q, m und n die unter Formel I angegebenen Bedeutungen besitzen ; oder

E) eine verbindung der Formel X

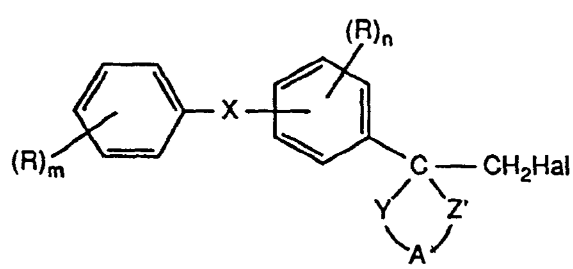

(X)

mit einer Verbindung der Formel XI

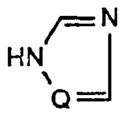

(XI),

worin R, X, Y, A, Q, m und n die unter Formel I angegebenen Bedeutungen besitzen, Z' den Rest $C(R_1)R_2$ darstellt und Hal Halogen bedeutet, in Gegenwart eines säurebindenden Mittels, in einem inerten organischen Lösungsmittel bei Temperaturen von 60° bis 160°C.

**19.** Mittel zum Schutz von Pflanzen gegen den Befall durch Mikroorganismen, dadurch gekennzeichnet, dass es neben üblichen Träger- und Hilfsstoffen als aktive Komponente mindestens eine Verbindung gemäss Anspruch 1 enthält.

**20.** Mittel gemäss Anspruch 19, dadurch gekennzeichnet, dass es als aktive Komponente mindestens eine Verbindung gemäss den Ansprüchen 2 bis 16 enthält.

**21.** Mittel gemäss Anspruch 19, dadurch gekennzeichnet, dass es als aktive Komponente eine Verbindung der Formel I gemäss Anspruch 17 enthält.

**22.** Verfahren zur Herstellung eines agrochemischen Mittels von Anspruch 19, dadurch gekennzeichnet, dass man mindestens eine gemäss Anspruch 1 definierte Verbindung mit geeigneten festen oder flüssigen Träger- und Hilfsstoffen innig vermischt.

**23.** Verwendung von Verbindungen gemäss Anspruch 1 zum Schutz von Pflanzen gegen den Befall durch phytopathogene Mikroorganismen.

**24.** Verwendung von Verbindungen gemäss einem der Ansprüche 2 bis 17 zum Schutz von Pflanzen gegen den Befall durch phytopathogene Mikroorganismen.

**25.** Verfahren zum Schutz von Pflanzen gegen den Befall durch phytopathogene Mikroorganismen, dadurch gekennzeichnet, dass man als Wirkstoff eine Verbindung gemäss Anspruch 1 auf die Pflanze oder deren Standort appliziert.

**26.** Verfahren zum Schutz von Pflanzen gegen den Befall durch phytopathogene Mikroorganismen, dadurch gekennzeichnet, dass man als Wirkstoff eine Verbindung gemäss einem der Ansprüche 2 bis 17 auf die Pflanze oder deren Standort appliziert.

**27.** Verfahren gemäss den Ansprüchen 25 und 26, dadurch gekennzeichnet, dass es sich bei den phytopathogenen Mikroorganismen um Pilzorganismen handelt.

Europäisches Patentamt

# EUROPÄISCHER RECHERCHENBERICHT

Nummer der Anmeldung

EP 91 81 0032

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| A | EP-A-171137 (UNIROYAL INC)<br>*Ansprüche 1-6; Tabelle 1, Verbindungen 8, 10, 11, 13* | 1, 18-27 | C07D417/06<br>C07D409/06<br>C07D411/06<br>C07D405/06<br>A01N43/653<br>A01N43/50<br>A01N43/72 |
| D | & AU-A-8541930<br>--- | | |
| A | EP-A-89100 (IMPERIAL CHEMICAL INDUSTRIES PLC)<br>* Ansprüche 1-8, 41-43 *<br>--- | 1, 18-27 | |
| A | EP-A-121979 (IMPERIAL CHEMICAL INDUSTRIES PLC)<br>* Ansprüche 1-8, 18-20 * | 1, 19-27 | |
| D | & EP-A-243983<br>--- | | |
| A | EP-A-92158 (MITSUBISHI CHEMICAL INDUSTRIES LTD)<br>* Ansprüche 1-13 *<br>--- | 1, 18-27 | |
| A | EP-A-224998 (UNIROYAL CHEMICAL COMPANY INC)<br>* Ansprüche 1-10 *<br>--- | 1, 18-27 | |
| A | EP-A-65485 (CIBA-GEIGY AG)<br>* Ansprüche 1-41, 46-49 *<br>--- | 1, 18-27 | RECHERCHIERTE SACHGEBIETE (Int. Cl.5) |
| A | DE-A-3505869 (CIBA-GEIGY AG)<br>* Ansprüche 1-20 *<br>----- | 1, 18-27 | C07D417/00<br>C07D409/00<br>C07D411/00<br>C07D405/00<br>C07D521/00<br>A01N43/00 |

Der vorliegende Recherchenbericht wurde für alle Patentansprüche erstellt

| Recherchenort | Abschlußdatum der Recherche | Prüfer |
|---|---|---|
| BERLIN | 06 MAI 1991 | VAN AMSTERDAM L. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)